(19) Europäisches Patentamt
European Patent Office
Office européen des brevets

(11) EP 2 539 431 B1

(12) EUROPEAN PATENT SPECIFICATION

(45) Date of publication and mention of the grant of the patent:
**06.04.2016 Bulletin 2016/14**

(21) Application number: **11717008.4**

(22) Date of filing: **11.03.2011**

(51) Int Cl.:
***C12N 1/14*** *(2006.01)*
***C12N 9/20*** *(2006.01)*
***C12N 9/42*** *(2006.01)*
***C12N 9/48*** *(2006.01)*
***C12N 9/58*** *(2006.01)*
***C12N 9/66*** *(2006.01)*
***C14C 1/06*** *(2006.01)*
***C11D 3/386*** *(2006.01)*
*C12R 1/645* *(2006.01)*

(86) International application number:
**PCT/IB2011/000516**

(87) International publication number:
**WO 2011/104630 (01.09.2011 Gazette 2011/35)**

(54) **ENZYMES FROM CONIDIOBOLUS BREFELDIANUS AND PROCESS FOR PREPARATION THEREOF**

ENZYME AUS CONIDIOBOLUS BREFELDIANUS UND HERSTELLUNGSVERFAHREN DAFÜR

ENZYMES DE CONIDIOBOLUS BREFELDIANUS ET PROCÉDÉ DE PRÉPARATION ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.02.2010 IN DE04372010**

(43) Date of publication of application:
**02.01.2013 Bulletin 2013/01**

(73) Proprietor: **Council of Scientific & Industrial Research**
**New Delhi 110 001 (IN)**

(72) Inventors:
- **RYALI, Seeta, Laxman**
  **411008 Pune**
  **Maharashtra (IN)**
- **KHANDELWAL, Harish, Bansilal**
  **411008 Pune**
  **Maharashtra (IN)**
- **MORE, Snehal, Vijay**
  **411008 Pune**
  **Maharashtra (IN)**
- **KALAL, Kamalakar, Motiram**
  **411008 Pune**
  **Maharashtra (IN)**
- **NARASIMHAN, Chandra, Babu, Kannan**
  **600020 Adyar**
  **Chennai (IN)**
- **PALANIVEL, Saravanan**
  **600020 Adyar**
  **Chennai (IN)**
- **BALARAM, Padmanabhan**
  **560012 Bangalore**
  **Karnataka (IN)**

(74) Representative: **Engelhard, Markus**
**Boehmert & Boehmert**
**Anwaltspartnerschaft mbB**
**Patentanwälte Rechtsanwälte**
**Pettenkoferstrasse 20-22**
**80336 München (DE)**

(56) References cited:
**GB-A- 804 608**
**US-A- 3 695 999**
**US-B2- 6 777 219**

- **Wioletta Wieloch: "Proteo-, chitino- and lipolytic enzymes production by entomopathogenic fungus Conidiobolus coronatus", Zjazd Polskiego Towarzystwa Biochemicznego, 2007, XP000002651805, Retrieved from the Internet: URL:http://science24.com/paper/10860 [retrieved on 2011-07-19]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- DATABASE BIOSIS [Online] BIOSCIENCES INFORMATION SERVICE, PHILADELPHIA, PA, US; 1984, LATGE J P ET AL: "AGGRESSIVENESS OF CONIDIOBOLUS-OBSCURUS AGAINST THE PEA APHID 3. EXOCELLULAR ENZYME ACTIVITIES", XP000002652470, Database accession no. PREV198579007667 & LATGE J P ET AL: "AGGRESSIVENESS OF CONIDIOBOLUS-OBSCURUS AGAINST THE PEA APHID 3. EXOCELLULAR ENZYME ACTIVITIES", ENTOMOPHAGA, vol. 29, no. 2, 1984, pages 185-202, ISSN: 0013-8959
- GABRIEL B P: "ENZYMATIC ACTIVITIES OF SOME ENTOMOPHTHOROUS FUNGI", JOURNAL OF INVERTEBRATE PATHOLOGY, vol. 11, no. 1, 1968, pages 70-81, XP55003092, ISSN: 0022-2011
- OKAFOR J I ET AL: "EXTRACELLULAR ENZYME ACTIVITIES BY BASIDIOBOLUS AND CONIDIOBOLUS ISOLATES ON SOLID MEDIA", MYKOSEN, vol. 30, no. 9, 1987, pages 404-407, XP000002651807, ISSN: 0027-5557
- MANNING ET AL: "Saprotrophy of Conidiobolus and Basidiobolus in leaf litter", MYCOLOGICAL RESEARCH, ELSEVIER, GB, vol. 111, no. 12, 1 December 2007 (2007-12-01), pages 1437-1449, XP022374045, ISSN: 0953-7562, DOI: 10.1016/J.MYCRES.2007.08.019
- DATABASE EMBL [Online] 21 October 2010 (2010-10-21), "Conidiobolus sp. RSL-II 18S ribosomal RNA gene, partial sequence.", XP000002651808, retrieved from EBI accession no. EMBL:FJ895304 Database accession no. FJ895304

## Description

### Field of invention

This invention relates to a fungal strain Conidiobolus brefeldianus bearing accession number MTCC-5185 and to a process for the preparation of a composition of enzymes comprising proteases, lipase, and carbohydrases from *Conidiobolus brefeldianus,* MTCC and to uses thereof.

### Background of invention and prior art

The current estimated value of worldwide sales of industrial enzymes is $1 billion. Of the industrial enzymes, 75% are hydrolytic. Proteases, amylases, lipases and xylanases together constitute around 85-90% total enzyme sales out of which proteases from plant, animal and microbial sources account for about 60%. Bacterial proteases are well known, fungal source of proteases are limited though advantageous. Further fungal source of proteases in combination with other enzymes are also limited.

*Conidiobolus coronatus* has been known to be used as source for the production of alkaline proteases, refer US6777219, US7186546, "Optimization and scale up of production of alkaline protease from Conidiobolus coronatus", by R. Seeta Laxman et.al; published in Process Biochemistry, Volume 40, Issue 9, Pages 3152-3158; dated September 2005, having doi:10.1016/j.procbio.2005.04.005 and "Thermostability of high-activity alkaline protease from Conidiobolus coronatus (NCL 86.8.20)" by S. H. Bhosale et.al; published in Enzyme and Microbial Technology 17:136-139, 1995. But there is no prior art for an enzyme composition that comprises proteases, lipases and carbohydrases from a strain of *Conidiobolus* species. US6777219 and US7186546 relate to processes for preparing alkaline proteases form *Conidiobolus coronatus.* Therefore the object of the invention is to provide an enzyme composition comprising of at least one enzyme selected from proteases, lipases, proteoglycanases, and carbohydrases from a fungal source, a hitherto unknown strain of *Conidiobolus brefeldianus*.

Another objective of the invention is to provide a process for preparation of an enzyme composition comprising of at least one enzyme selected from proteases, lipase, and carbohydrases using a fungal culture *Conidiobolus brefeldianus* isolated by the inventors.

Another objective of the present invention is to provide a process for the preparation of alkaline protease which is active and stable in wide pH range and in short fermentation cycles.

Still another objective of the present invention is to provide an economical process for the production of proteases, lipase, and carbohydrases alone or in combinations thereof. The object of the present invention is also to disclose the process of preparation of proteases, lipase, proteoglycanases, and carbohydrases alone or in combinations thereof on inexpensive media by a fungal culture belonging to the genus *Conidiobolus brefeldianus* isolated at National Chemical Laboratory, Pune, India.

Yet another objective of the invention is to provide methods of use of the enzymes of the invention in leather, detergent, food, textile, degumming of silk, production and recovery of serecin proteins/peptides from silk/silk wastes, animal tissue culture, analytical tools, pharmaceutical, cosmetics, molecular biology, and such like industries.

### Summary of the invention:

Accordingly, the present invention provides a fungal strain Conidiobolus brefeldianus with accession number MTCC-5185 and a process for preparing an enzyme composition comprising at least one enzyme selected from protease, lipase, and carbohydrase from a fungal source, a hitherto unknown strain of *Conidiobolus brefeldianus* MTCC-5185. The process of preparation of the enzyme composition and uses thereof are as disclosed in the appended claims. The enzyme composition of the invention comprises the enzymes disclosed herein alone or in combinations thereof. The proteases of the invention are selected from, but not limited to alkaline proteases, proteases, elastase, keratinase, and peptidase, alone or in combinations thereof.

The carbohydrases of the invention are selected from, but not limited to glycosidases, particularly glycanases, more particularly chitinase, laminarinase, chondrotinase, alone or in combinations thereof.

The lipases of the invention comprise esterases.

In one embodiment, the invention relates to enzyme compositions comprising alkaline proteases that are active, stable over a wide pH range, stable to chemicals, temperature changes and have a shelf life as disclosed herein. The composition of the invention is stable to chemicals selected from, but not restricted to detergents, organic solvents, denaturants and similar compounds, having applications in leather, cosmetics, textile, food, detergent, pharmaceutical and other industries.

In one more embodiment of the invention, the enzymes composition is in the form of biomass

In another embodiment of the invention the enzyme composition comprises enzymes in crude form.

In another embodiment of the invention the enzyme composition comprises enzymes in purified form.
In another embodiment of the invention the enzyme composition comprises enzymes in refined form.
In yet another embodiment of the invention the enzyme composition comprises enzymes in free form.
In yet another embodiment of the invention the enzyme composition comprises enzymes in combination of free and immobilized form.
In yet another embodiment of the invention the enzyme composition comprises enzymes in immobilized form.
In yet another embodiment of the invention the enzyme composition comprises enzymes in cell bound form.
In another embodiment of the invention, the enzyme composition comprises enzymes in intra cellular form.
The present invention provides a fermentation process for preparation of one or more enzymes selected from the group consisting of protease, carbohydrase and lipase either singly or simultaneously from *Conidiobolus brefeldianus* depending on the carbon, nitrogen and inducer used, wherein the said process comprising of:

a. growing the fungal strain *Conidiobolus brefeldianus* MTCC 5185 in a medium comprising a carbon and a nitrogen source and an inducer under aerobic conditions, pH ranging from 5.0 to 10.0, and temperatures ranging between 15° to 37°C, for periods ranging between 2 to 5 days;
b. harvesting the medium and
c. separating/extracting the enzyme in liquid phase by conventional methods.

In an embodiment of the process, the organism is grown in submerged culture with shaking at 180 to 220 rpm.
In another embodiment, the organism is grown in semi-solid culture under stationary conditions.
The carbon sources comprise of, but not restricted to sugars, sugar alcohols, polysaccharides, agricultural products, agricultural by products/wastes and such like, alone or in combinations thereof. The sugars are glucose, fructose, arabinose, sucrose, lactose and such like; sugar alcohols are glycerol, mannitol, sorbitol and such like; polysaccharides are starch and such like oils/fats are olive oil, sunflower oil, soyabean oil, gingelly oil, mustard oil, castor oil, coconut oil ground nut oil, tributyrin and such like and agricultural products/wastes are soya flour, soyabean meal, ground nut meal, mustard seed cake, cotton seed cake, wheat bran, rice bran and such like; chitin containing wastes like crab shells and such like.
The nitrogen source is optionally organic or inorganic, alone or in combinations thereof. The inorganic nitrogen sources are selected from, but not limited to di-ammonium hydrogen phosphate, ammonium sulphate, ammonium chloride, sodium nitrate, potassium nitrate and urea.
The organic nitrogen sources are selected from, but not limited to peptone, tryptone, soyatose, soyapeptone, casamino acids, casein, meat extract, beef extract, yeast extract, fish meal, feather meal, feathers, corn steep liquor and nitrogen-rich leguminous substrates exemplified by soya flour, soyameal, gram flour, mung flour, mustard seed cake, cotton seed cake, ground nut meal, wheat bran, rice bran and such like, wastes from dairy, poultry, meat and food processing, keratin rich wastes like hair, feathers, wastes from fisheries and other wastes, alone or in combinations thereof.
The conventional methods of separating enzymes are filtration, centrifugation or extraction with water or dilute surfactants. In an embodiment of the invention, the enzymes in intra cellular form re separated by including steps of cell breakage by sonication and mechanical grinding.
Carbon and nitrogen sources/ inducers are selected from, but not limited to protein/nitrogen containing wastes from dairy like whey, food processing, meat and fish exemplified by fish meal and chicken feathers, feather meal and such like, or agricultural wastes, exemplified as oil seed cakes, and carbohydrate wastes like waste cereals/grains, oils, fats, tannery wastes like fleshings, trimmings and chrome shavings, keratin rich substrates like nails, hoofs, hair alone or in combinations thereof
In an embodiment of the invention, the organic nitrogen sources are inducers for protease.
In a feature of the present invention, concentration of the enzymes was achieved either by membrane filtration or by salting out through addition of salts such as ammonium sulphate, sodium sulfate, sodium chloride, magnesium chloride etc., addition of organic solvents such as ethanol, acetone or by freeze drying or spray drying.
In a feature of the present invention, the lyophilized protease is stable at temperatures ranging from 4 to 40°C.
In a feature of the present invention, the spray dried protease is stable at temperatures ranging from 4 to 40°C (760 days)
The protease of the invention is stable at pH range of 3-12, preferably pH 7, stable in presence of detergents, water miscible and water immiscible organic solvents, and stable up to 50°C. The protease is active in the pH range of 6-11, temperature range 30 to 60°C and in presence of chelators, metal ions.
In an embodiment of the invention, the crude as well as partially purified protease show activity towards casein, albumin, haemoglobin, keratin, elastin-orcin, azocasein, azocoll, N-α-benzoyl-DL-arginine-p-nitroanilide (BAPNA) and gelatin.
In another embodiment of the invention, the protease was serine protease and inhibited by phenyl methyl

sulphonyl fluoride (PMSF).

In an embodiment of the invention, lipase is active in the pH range of 4 to 9 preferably at pH 7 and temperature range of 20 to 60°C preferably at 50°C.

In another embodiment of the invention, the enzyme composition is inert to true collagen.

In another embodiment of the invention, the protease is useful in degumming of silk and recovers serecin protein/peptides from degummed waste liquor.

The enzyme composition of the invention finds use in food, cosmetic, pharmaceutical, leather, textile industries, and resolution of optical isomers from racemic mixtures. Proteases find application in agriculture, leather, textile, degumming of silk, dairy, food, feed, detergent, pharmaceutical industries, animal tissue culture, silver removal from waste photographic films and nanoparticle synthesis, fertilizer, preparation of media ingredients and protein hydrolysates from plant, animal, peptide synthesis, in molecular biology, cosmetics, separation of racemic mixture of amino acids, waste treatment and such like. Keratinases find application in leather, detergent, prior degradation in mad cow disease and such like. Lipases find application in agriculture, leather, textile, dairy, food, feed, detergent, pharmaceutical industries, fertilizer, cosmetics, waste treatment, polymer synthesis and such like. Chitinases find application in agriculture. Chondrotinase finds application in spinal cord injury.

**Detailed description of drawings:**

Fig.1 Microscopic picture of *Conidiobolus brefeldianus* MTCC 5185 (a) fungal mycelia showing protoplasmic contents, (b) thick walled zygospores with oil droplets

Fig.2: Dendrogram showing homology on the basis of multiple sequence alignment of 18S rDNA sequences. 18S rDNA sequence of new fungal strain MTCC 5185 shows maximum homology with *Conidioblous brefeldianus.*

Fig. 3: Plate assay showing production of lipase by *Conidiobolus brefeldianus* MTCC 5185. Clearance zone around fungal colonies on tributyrin agar plate after five days of growth indicating lipase secretion by the fungus.

Fig. 4: Plate assay showing production of chitinase by *Conidiobolus brefeldianus* MTCC 5185. Clearance zone on acid swollen chitin plate around the well where culture filtrate was added indicates secretion of chitinase by the fungus.

Fig.5: Plate assay showing production of chondroitinase by *Conidiobolus brefeldianus* MTCC 5185. Clear zone around the colony on Chondroitin sulphate A plate after 4 days of growth indicates the secretion of chondroitinase by the fungus.

Fig. 6: SDS-PAGE of purified protease from *Conidiobolus brefeldianus* MTCC 5185 showing single peak which corresponds to the molecular weight of around 29.9kDa.

Fig.7: MALDI - TOF of partially purified protease showing a major peak with molecular weight 27.8 kDa

Fig 8: Shelf life of spray dried protease .Spray dried protease is stable for more than two years at temperatures up to 40°C.

Fig.9: Removal of silver containing gelatin layer from waste x-ray film by *Conidiobolus brefeldianus:* $\alpha$-crude protease; b-biomass filtrate and c-biomass. The solution turned blackish due to gelatin removal from the film and simultaneous release of silver in to the solution. After 1 h, the film is clean due to complete removal of silver and gelatin.

Fig 10: Visible observation of silk fiber before and after degumming with protease.

Luster of silk fiber is seen after degumming with protease.

Fig. 11: Scanning electron micrographs of silk fiber before and after degumming with protease. Silk fiber is smooth due to serecin removal after degumming.

Fig 12: Serecin recovery in waste liquor after enzymatic degumming with *Conidiobolus brefeldianus* 5185 protease. Insoluble residue of serecin in the degumming liquor increased with increase in protease concentration used for degumming.

Fig. 13: SDS-PAGE of serecin peptides recovered from waste degumming liquid after enzymatic degumming with *Conidiobolus brefeldianus* 5185 protease.

Fig. 14: Figure showing removal of blood stains. Crude protease was able to remove blood stain along with blood clots while commercial detergent removed only stains. a-water; b-detergent; c-protease; d-protease+ detergent

**Detailed description of the invention**

An enzyme composition comprising at least one enzyme selected from, but not limited to protease, lipase, carbohydrase enzyme with hemagglutiriation activity and glycoprotein degrading activity from a fungal source, a hitherto unknown strain of *Conidiobolus brefeldianus* MTCC-5185 is disclosed herein. The process of preparation of the enzyme composition and uses thereof are also disclosed. The enzyme composition of the invention comprises the enzymes disclosed herein alone or in combinations thereof.

The fungal strain, *Conidiobolus brefeldianus* is isolated from plant detritus and bears the accession number MTCC-5185 (Microbial Type Culture Collection, Chandigarh, India). The sources of *Conidiobolus brefeldianus* are soil, insect, leaf litter, tree twigs, and such like.. Fig 1 shows the microscopic picture of the fungal mycelia of *Conidiobolus brefeldianus* MTCC 5185 with protoplasmic contents and the thick walled zygospores with oil droplets. The Fig. 2 shows the sequence homology of 18S rDNA gene with the other organisms. According to the 18S rDNA sequence homology, the isolated strain-of the invention shows 99% homology to the *Conidiobolus brefeldianus* strain AF368506.1 and 98% homology to different strains of *Conidiobolus coronatus*. The 18S rDNA gene sequence of MTCC 5185, a new strain of *Conidiobolus brefeldianus* has been deposited with NCBI gene bank with the accession number FJ895304.

The proteases of the invention are selected from, but not limited to alkaline proteases, proteases, elastase, keratinase, and peptidase, alone or in combinations thereof.

The carbohydrases of the invention are selected from, but not limited to glycosidases, particularly glycanases, more particularly chitinase, laminarinase, chondrotinase, alone or in combinations thereof.

The lipases of the invention comprise esterases.

The determination of enzyme activity has been carried out by methods as described herein. The reaction mixture contained an aliquot of suitably diluted enzyme solution and 10mg Hammerstein casein in 0.1 M sodium carbonate buffer pH 9.0 in a total volume of 2 ml. After incubation at 50°C for 10 min, the reaction was terminated by the addition of 3 ml of 5% trichloroacetic acid (acidified with concentrated hydrochloric acid). The precipitate formed was filtered through Whatman No.1 filter paper after standing for 30 min at room temperature. The absorbance of trichloroacetic acid soluble fraction was measured at 280 nm. Micrograms of tyrosine produced was calculated from a pre-calibrated graph of absorbance at 280 nm against tyrosine concentration and the units are expressed as μmoles of tyrosine released per minute under assay conditions.

The laminarinase (β-1, 3 glucanase) activity was measured using 1% laminarin in 50mM potassium phosphate buffer pH 7. The total reaction mixture of 1ml contained 0.5 ml of suitably diluted enzyme and 0.5ml substrate and incubated at 50°C for 30 min. The reducing sugar liberated was measured by dinitro salicylic acid method (P. Bernfeld 1955, Amylase: α & β, Methods in Enzymology, Volume 1, 149). Laminarinase activity was expressed as μmoles of reducing sugar (as glucose equivalents) produced per min under the assay conditions.

Lipase activity was measured by two different methods.

a. Spectrophotometric assay using p-nitro phenol butyrate (pNPB) as substrate: 30mg pNPB was dissolved in 10ml of isopropanol and 0.1ml Triton-X-100 and volume made to 100 ml with 50 mM phosphate buffer, pH 7. The assay mixture contained 0.9ml substrate and 0.1ml suitably diluted enzyme. The assay mixture was incubated at 50°C for 30min and terminated by addition of 2ml of isopropanol. The amount of p-nitrophenol released was measured at 410nm. The lipase activity is expressed as μmoles of p-nitro phenol released per 30 min under the assay conditions.

b. Titrimetric assay of Lipase: The substrate was prepared by mixing 20ml of olive oil, 165 ml of 10% gum arabic and 15g ice in grinder mixer for 10 minutes and filtered on glass wool and stored at 4°C. For lipase assay the reaction mixture contained 2 ml phosphate buffer (50 mM, pH 7.0), 5 ml substrate and 1 ml crude culture broth and incubated at 50°C for 1 h with shaking at 50 rpm. The reaction was terminated by addition of 4 ml of acetone:ethanol (1:1). In blank the enzyme was added after the termination of reaction by acetone:ethanol. Free fatty acids released were titrated with 10 mM NaOH. The lipase activity is expressed as μmoles of free fatty acids released per min under the assay conditions.

Chitinase assay was measured using 0.7% chitin. Reaction mixture contained 1 ml of enzyme, 1 ml of 50mM acetate buffer, pH 5 and 1 ml of 0.7% chitin as substrate. The reaction mixture was incubated at 50°C for 1 h. The N-acetylglucosamine liberated was estimated by measuring the absorbance at 585 nm spectrometrically with p-dimethyl amino benzaldehyde. One unit of activity is defined as the amount of enzyme required to liberate 1 μ mole of N-acetyl glucosamine per minute under the assay conditions.

Chondroitinase activity was measured by spectrophotometric method at 37°C. Appropriately diluted enzyme (0.8 ml) was equilibrated at 37°C for 10 min after which 0.2 ml of 0.5% Chondroitin sulphate A in 0.05% bovine serum albumin prepared in 250 mM Tris HCl and 300 mM sodium acetate buffer, pH 8 was added and mixed immediately. The reaction mixture was incubated at 37°C up to 21 min. Aliquots of 0.1 ml were withdrawn at an interval of 3 min and transferred to tubes containing 0.9 ml of 50 mM KCl adjusted to pH 8 and incubation continued at 37 °C for another 10 min. At the end of 10 min, the contents were centrifuged and absorbance was measured at 232 nm. Absorbance at zero minute served as blank for the assay. Activity was calculated from the slope (increase in absorbance/per min) of the liner portion of the graph of absorbance against time as shown below. One unit is defined as the amount of enzyme required to liberate one micromole of 2-acetamido-2-deoxy-3-O-(ß-D-gluc-4-ene-pyranosyluronic acid)-4-O-sulfo-D-galactose from chondroitin sulfate A per minute under the assay conditions.

$$U/ml = \frac{\text{Slope} \times 1\ (\text{ml}) \times \text{dilution factor}}{5.1\ (\text{EmM}) \times 0.1 \times 0.8}$$

where 5.1 is the milli molar extinction coefficient of unsaturated disaccharides for chondroitin sulfate A

Traditionally, keratinases have been used for production of feather meal, fertilizers and glues etc. Their application range is slowly widening and now they are increasingly being used in to other areas such as detergent formulation, cosmetics, leather, medicine and animal feed. More recently, they find applications in treatment of mad cow disease (degradation of prion), biodegradable plastic and feather meal production. Application of keratinases having mild elastolytic activity but lacking collaginolytic is being explored for the dehairing process in leather manufacture. Skins and hides contain substantial quantities of valuable GAGs (0.2-0.3% on raw weight. A n enzyme composition comprising of lipase, keratinase, chondrotinase and chitinase will be useful dehairing.

The enzyme composition of the invention finds use in food, cosmetic, pharmaceutical, leather, textile industries, and resolution of optical isomers from racemic mixtures. Proteases find application in agriculture, leather, textile, degumming of silk, dairy, food, feed, detergent, pharmaceutical industries, animal tissue culture, silver removal from waste photographic films and nanoparticle synthesis, fertilizer, preparation of media ingredients and protein hydrolysates from plant, animal, peptide synthesis, in molecular biology, cosmetics, separation of racemic mixture of amino acids, waste treatment and such like. Keratinases find application in leather, detergent, prior degradation in mad cow disease and such like. Lipases find application in agriculture, leather, textile, dairy, food, feed, detergent, pharmaceutical industries, fertilizer, cosmetics, waste treatment, polymer synthesis and such like. Chitinases find application in agriculture. Chondrotinase finds application in spinal cord injury.

The present invention is described herein below with examples, which are illustrative only and should not be construed to limit the scope of the present invention in any manner whatsoever.

**The present invention is described herein below with examples, which are illustrative only and should not be construed to limit the scope of the present invention in any manner whatsoever.**

EXAMPLES

Example 1

This example illustrates the isolation of the *Conidiobolus brefeldianus* MTCC 5185 and its identification by morphological characteristics. The fungal culture was isolated from decomposing plant detritus collected from Pune, Maharashtra, India. Fine particles of plant detritus were superimposed on MGYP agar (malt extract-0.3%; yeast extract-0.3%; peptone-0.5%, gluscose-1%, agar-2%) blocks attached to the inner surface of the petri plate lid and plates were incubated at 28°C. Isolated single colonies developing from forcibly discharged conidia were picked up and transferred to MGYP agar slants, incubated at 28°C for 2-3 days. The organism grows rapidly and was identified as a strain belonging to genus *Conidiobolus* on the basis of the morphology of the forcibly discharged large globose conidia with basal papillae. Mycelium is coenocytic but becomes septate in later stages. Conidiophores are micronemous and indistinguishable from mycelium. At the tip of the conidiophore, large globose conidia are formed which are discharged forcibly. The size of conidia varies between 35-45 microns. Conidia either germinate to give rise to mycelium or to microconidia on radial sterigmata or to succession of secondary conidia. The discharged conidia are visible as whitish/creamish deposit on the glass above the growing culture. The organism is zygosporic and the zygospores (resting spores) are round, smooth and thick walled with 2 distinct wall layers and granular contents inside (Fig. 1).

**Example 2A**

This example illustrates the isolation of genomic DNA of *Conidiobolus brefeldianus* MTCC 5185 by cetyl trimethyl ammonium bromide (CTAB) method. For isolation of DNA, the fungus was grown in liquid flasks in malt extract glucose yeast extract peptone (MGYP) medium the composition of which is (g/L) malt extract-3; yeast extract-3; peptone-5 and glucose-10. Growth was initiated by inoculating spores from a 7 days old MGYP slant. The flasks were incubated on a rotary shaker (200rpm) for 48 hours at 28°C. The contents were centrifuged at 8000 rpm for 15min, washed repeatedly to remove the media constituents. 3-5 g of wet mycelium was ground in liquid nitrogen, followed by addition of 8-10 ml of CTAB extraction buffer, pH 8 containing 0.2% β-mercaptoethanol after which 20μl of proteinase K (20mg/ml) was added and incubated at 65°C for 1h. This was followed by addition of 20μl RNase A (10mg/ml) and further incubation at 65°C for 15min. To the supernatant collected after centrifugation (8000rpm, 10min), 10ml chloroform: isoamylalcohol (24:1) was added. The mixture was shaken for 5min and centrifuged at 10,000rpm, 4°C for 15min. Two volumes of CTAB precipitation buffer, was added to the supernatant and kept at room temperature for 1h. The pellet collected after

centrifugation was dissolved in 5ml of 1.2 M NaCl and 5ml of chloroform: isoamylalcohol (24:1) was added. Two volumes of absolute alcohol were added to the aqueous phase to precipitate the DNA. DNA was spooled out and washed with 70% ethanol and dissolved in 5ml of 0.1M Tris EDTA buffer pH 8.0 and stored. The quantification of DNA was done by measuring the absorbance of the sample at 260nm on spectrophotometer and purity was checked on 0.8 % agarose gel electrophoresis.

**Example 2B**

This example illustrates the polymerase chain reaction (PCR) amplification of genomic DNA for 18S rDNA gene. The primers used for the identification of fungal species were universal fungal 18S ribosomal DNA (rDNA) primers NS1-F (GTA GTC ATA TGC TTG TCT C), NS8-R (TCC GCA GGT TCA CCT ACG GA). The polymerase chain reaction (25μl) was set to amplify the 18S rDNA gene by using the genomic DNA. The reaction mixture typically contained genomic DNA-0.70μl, 10X PCR Buffer-2.50μl, 0.2mM dNTPs-2.5μl, forward and reverse primers 10-20 pmoles-1.25μl each, distilled waster-16.60μl, and 1unit of *Taq* DNA polymerse-0.20μl. The PCR conditions for 18S rDNA gene amplification were: initial denaturation- 95°C for 3min; followed by 35 cycles of 94°C for 1min, 57°C for 30sec, 72°C for 2min and final extension at 72°C for 10min. 5μl of the above PCR amplified product was used to check the amplification on 1.0% agarose gel.

**Example 2C**

This example illustrates the purification of PCR amplified products. To 20μl PCR amplified products, 12μl of 20% PEG-NaCl (Polyethylene glycol - NaCl) solution was added and incubated at 37°C for 30min. It was then centrifuged at 12,000rpm for 20min. The supernatant was discarded and the pellet was washed twice with 70% ethanol and separated by centrifuging at 12,000rpm for 20min. The pellet was dried and dissolved in 10μl of double distilled water and stored at -20°C.

**Example 2D**

This example illustrates the sequencing of the purified PCR products. The sequencing reactions were carried out using *Taq* DNA polymerase using the 'ABI PRISM BigDye Terminator Cycle Sequencing Ready Reaction Kit' (Perkin Elmer Applied Biosystems Division, Foster City, CA) according to the manufacturer's protocol. This Kit contains the four ddNTPs with different fluorescence labels termed as BigDye Terminators. 2μl PCR product and 3pmol of the sequencing primer were used in a 20μl sequencing reaction. The sequencing primers were NS1 (GTA GTC ATA TGC TTG TCT C), NS2 (GGC TGC TGG CAC CAG ACT TGC), NS3 (GCA AGT CTG GTG CCA GCA GCC), NS4 (CTT CCG TCA ATT CCT TTA AG), NS5 (AAC TTA AAG GAA TTG ACG GAA G), NS6 (GCA TCA CAG ACC TGT TAT TGC CTC), NS7 (GAG GCA ATA ACA GGT CTG TGA TGC) and NS8 (TCC GCA GGT TCA CCT ACG GA) for sequencing ( White et al 1990). The sequencing reaction mixes were subjected to 25 cycles in a Perkin Elmer thermal cycler 9700. Each cycle consisted of 95°C for 10min, 50°C for 5min and 60°C for 4min. DNA sequencing was carried out on ABI 1500 Automated Sequencer.

**Example 2E**

This example illustrates the identification and phylogenetic relationship of new strain of *Conidiobolus* sp MTCC 5185 on the basis of 18S rDNA sequence obtained in above example. The sequences obtained were in small fragments and hence it was aligned properly by overlapping the sequences. Percentage homology and phylogenetic relationship of new strain of *Conidiobolus* sp using obtained sequences of 18S rDNA were done in NCBI database. The nucleotide sequence was analyzed with the GenBank database using Basic Local Alignment Search Tool (BLAST) program (www.ncbi.ncm.gov/blast). The result in the Table 1 shows the first 10 BLAST hit of 18S rDNA sequence in NCBI database. 18S rDNA showed maximum (99%) sequence homology with one strain of *Conidiobolus brefeldianus* AF 368506.1. The 18S rDNA sequence of MTCC 5185, a new strain of *Conidiobolus brefeldianus* has been deposited in NCBI gene bank with the accession number FJ895304. The Figure 2 shows the sequence homology of 18S rDNA with the other organisms. It can be seen that strain *Conidiobolus brefeldianus* MTCC 5185 does not fall in the *Conidiobolus coronatus* cluster.

Table 1: First 10 BLAST hit of 18S rDNA

| Sequences showing significant alignment | | Score (Bit) | E value | Homology |
|---|---|---|---|---|
| AF368506.1 | *Conidiobolus brefeldianus* small subunit ribosomal RNA gene, partial sequence | 3000 | 0.0 | 99% |
| AF113417.1 | *Conidiobolus coronatus* strain NRRL1912 18S ribosomal RNA gene, partial sequence | 2922 | 0.0 | 98% |
| D29947.1 | *Conidiobolus coronatus* gene for 18S rRNA | 2922 | 0.0 | 98% |
| AF296753.1 | *Conidiobolus coronatus* small subunit ribosomal RNA gene, partial sequence | 2905 | 0.0 | 98% |
| AF368507.1 | *Conidiobolus firmipilleus* small subunit ribosomal RNA gene, partial sequence | 2900 | 0.0 | 98% |
| AF113418.1 | *Conidiobolus coronatus* strain NRRL28638 18S ribosomal RNA gene, partial sequence | 2900 | 0.0 | 98% |
| AF113419.1 | *Conidiobolus incongruus* 18S ribosomal RNA gene, partial sequence | 2776 | 0.0 | 96% |
| EF392543.1 | *Conidiobolus coronatus* strain ARSEF 206 18S ribosomal RNA gene, partial sequence | 1724 | 0.0 | 98% |
| AF368512.1 | *Conidiobolus rhysosporus* small subunit ribosomal RNA gene, partial sequence | 1387 | 0.0 | 82% |
| AF368511.1 | *Conidiobolus pumilus* small subunit ribosomal RNA gene, partial sequence | 1343 | 0.0 | 82% |

**Example 3**

This example illustrates the preparation of the protease by submerged fermentation using the strain *Conidiobolus brefeldianus* MTCC 5185. Fermentation was carried out in shake flasks in a medium containing (grams per liter) Glucose-20; Yeast extract- 3, Soyabean meal -30. Spores from a 3 days old MGYP slant were used for preparing the seed culture, the composition of which is (grams per liter) Glucose-10; Yeast extract- 3. This was incubated on a rotary shaker for 24 hours at 28°C and was used to initiate the shake flask experiments. The shake flask experiment was run for 72 h at 28°C on a rotary shaker (200 rpm). Protease activity was estimated according to Laxman et al (2005), Process Biochemistry, Volume 40, 3152-3158 (2005). One unit of activity is defined as the amount of enzyme required to release one micromole of Tyr/min. The protease activity in the cell free broth after 3 days was 36-38 IU/ml.

**Example 4**

This example illustrates the preparation of the protease by submerged fermentation using the strain *Conidiobolus brefeldianus* MTCC 5185. Fermentation was carried out in shake flasks in a medium containing (grams per liter) Glucose-20; Di-ammonium hydrogen phosphate - 1.6, Soybean meal -30. Spores from a 3 days old MGYP slant were used for preparing the seed culture, the composition of which is (grams per liter) Glucose-10; Yeast extract- 3; Peptone-5. This was incubated on a rotary shaker for 24 hours at 28°C and was used to initiate the shake flask experiments. The shake flask experiment was run for 72 h at 28°C on a rotary shaker (200 rpm). The activity in the cell free broth after 3 days was 34 -40 IU/ml.

**Example 5**

This example illustrates the preparation of the protease by submerged fermentation in an instrumented fermentor under controlled conditions of agitation and aeration using the strain *Conidiobolus brefeldianus* MTCC 5185. Fermentation was carried out in 7.5 L New Bruinswick fermentor with 5 L working volume. Spores from 3 days old MGYP plate/slant were used to develop the inoculum in 1 L flasks containing 250ml medium the composition of which is (grams per liter) yeast extract-3; peptone-5 and glucose-10 (GYEP) and incubated for 24 h at 28°C, 220 rpm. The fermentor was inoculated with 500ml (10% v/v) of 24 h vegetative inoculum. The production medium (4.5 L) in 7.5L fermentor contained 2% commercial grade glucose, 0.3% yeast extract as a basal medium. Soyabean meal at a concentration of 3% was used

as an inducer. Aeration and stirrer speed were kept at 3-4 rpm and 350-400 rpm respectively and temperature was maintained at 26-28°C. The activity in the cell free broth after 2-3 days was 36-40 IU/ml.

**Example 6**

This example illustrates the preparation of the protease by submerged fermentation in an instrumented fermentor under controlled conditions of agitation and aeration using the strain *Conidiobolus brefeldianus* MTCC 5185. Fermentation was carried out in 75 L fermentor with 50 L working volume. Following fermentation parameters were followed for production: 3 days old MGYP slant as stock; 15h old pre-inoculum grown in medium the composition of which is (grams per liter) yeast extract-3; peptone-5 and glucose-10 (GYEP); 15h old inoculum grown in medium the composition of which is (grams per liter) yeast extract-3 and glucose-10 (GYE); production medium containing 2% commercial glucose, 0.16% fertilizer grade di-ammonium hydrogen phosphate (DAP) and 3% Soyabean meal (SBM). Initially, SBM along with antifoam were sterilized at 121°C for 30min. After cooling, glucose and DAP were added and resterilized for 20min and kept under positive pressure until inoculation. Temperature was maintained at 28-30°C throughout the fermentation. Agitation was kept initially at 80 rpm and increased slowly to a maximum of 120 rpm at the end the fermentation. Aeration was kept initially at 0.8wm and increased to 1.2-1.4vvm after 36h. The activity in the cell free broth after 2-3 days was 36-40 IU/ml.

**Example 7**

This example illustrates the detection of lipase activity secreted by *Conidiobolus brefeldianus* MTCC 5185 using plate assay. Plate assay for enzyme was carried out in disposable petri-plate containing 25 ml Mikami medium which was composed of (grams per liter): glucose-1.5; yeast extract-1.5; peptone-5; beef extract-5, agar-20 and 1% emulsified tributyrin. Spore suspension from a 2 day old slant was used for preparing the GYE inoculum whose composition was (grams per liter): yeast extract-3 and glucose-10. After 24 hours of growth, one loopful of growth was spot inoculated on the plate and incubated at 28° C for 72 h. A zone of clearance around the growing fungal colonies due to the degradation of tributyrin was observed indicating secretion of lipase by the organism (Fig. 3).

**Example 8**

This example illustrates the detection of chitinase activity using plate assay by the strain *Conidiobolus brefeldianus* MTCC 5185. Plate assay was carried out in 25 ml of 2% agar containing 0.01% acid swollen chitin in disposable petri-plate. Spores from a 3 day old slant were inoculated in the production medium the composition of which is (grams per liter): yeast extract-3; glucose-10 and acid swollen chitin-0.1 and incubated at 28°C with shaking at 180-200rpm. Samples were removed after 24, 48 and 72 h and 50 $\mu$l of cell free supernatant was added in the wells made on the plate and incubated at 37°C for 1 h. For detection of chitinase activity, the plate was flooded with 0.1% ranipal for 15 min followed by washing twice with distilled water for 30 min each. The plate was observed under UV light. A light blue clear zone around the sample spot was observed due to chitin degradation indicating secretion of chitinase by the fungal strain (Fig. 4). There was no clearance zone where sterile water was added (well 1) while, zone of clearance increased with fermentation time and maximum clearance was observed in 72 h sample (well 4).

**Example 9**

This example illustrates the detection of chondroitinase activity secreted by the strain *Conidiobolus brefeldianus* MTCC 5185 using plate assay. Plate assay for enzyme was carried out in disposable petri-plate containing MGYP agar medium supplemented with bovine serum albumin (BSA) and Chondroitin sulphate A. Malt extract-0.15g; yeast extract-0.15g; peptone-0.25g; glucose-0.5g were dissolved in 40ml distilled water, 1g agar was added and autoclaved. BSA (500mg) and Chondroitin sulphate A (20mg) were dissolved in 10ml) distilled water and filter sterilized and added to the medium and poured in sterile petri plates. Spore suspension from a 2 day old slant was used to inoculate GYE medium whose composition was (grams per liter): yeast extract-3 and glucose-10. After 24 hours of growth, 10% (v/v) vegetative growth was transferred to the medium containing (grams per liter): glucose-20; diammonium hydrogen phosphate-1.6; soyabean meal-30 and incubated at 28°C, 180 rpm. After 48h, one loopful of growth was spot inoculated on the plate and incubated at 28° C. After four days, the plate was flooded with 2N acetic acid and allowed to stand for 15 minutes at room temperature. Un-degraded chondroitin sulphate A conjugated with BSA giving opaque appearance while a zone of clearance around the colony was seen indicating the secretion of chondrotinase leading to hydrolysis of chondroitin sulphate A. (Fig. 5).

**Example 10**

This example illustrates the preparation of the **lipase** by submerged fermentation using the strain *Conidiobolus brefeldianus* MTCC 5185. Spore suspension from a 2 day old slant was used for preparing the glucose yeast extract seed culture, the composition of which is (grams per liter): yeast extract-3, glucose-10. This was incubated on a rotary shaker at 28° C for 24 hours and was used to initiate the shake flask experiments. Fermentation was carried out in shake flasks in Mikami medium containing (grams per liter): peptone-5; beef extract-5; yeast extract-1.5; glucose-1.5. Olive oil at 2% concentration was used as inducer for lipase production. Medium was adjusted to pH 7. Lipase activities in the cell free broth after 3 days were determined spectrophotometric method using p-nitrophenyl butyrate as substrate and activities were in the range of 0.5-.0.6 IU/ml.

**Example 11**

This example illustrates the inductive effect of various oils and fats on production of the lipase by submerged fermentation using the strain *Conidiobolus brefeldianus* MTCC 5185. Spore suspension from a 2 day old slant was used for preparing the glucose yeast extract seed culture, the composition of which is (grams per liter): yeast extract-3, glucose-10. This was incubated on a rotary shaker at 28° C for 24 hours and was used to initiate the shake flask experiments. Fermentation was carried out in shake flasks in Mikami medium containing (grams per liter): peptone-5; beef extract-5; yeast extract-1.5; glucose-1.5. Following oils at 2% concentration were used as inducers for lipase production: soyabean oil; olive oil, sunflower oil and tributyrin. Medium was adjusted to pH 7. Lipase activities in the cell free broth after 4 days were determined by titrimetric method. Lipase activities in soyabean; olive and sunflower oils ranged between 0.41-0.43 U/ml while tributyrin gave higher activities of 0.63 U/ml.

**Example 12**

This example illustrates the preparation of the **protease** and **lipase** by submerged fermentation using the strain *Conidiobolus brefeldianus* MTCC 5185. Spore suspension from a 2 day old slant was used for inoculating the fermentation medium whose composition is (grams per liter): Malt extract-3; peptone-5., glucose-10; yeast extract-3. Medium was adjusted to pH 7. Soyabean meal (2%) and soyabean oil (1%) were used as inducers for protease and lipase production respectively. Protease and lipase activities in the cell free broth after 3 days were determined by caseinolytic and titrimetric methods respectively. Protease and lipase activities were in the range of 12-15 IU/ml and 1-1.2 IU/ml respectively.

**Example 13**

This example illustrates the preparation of the **chitinase** by submerged fermentation using the strain *Conidiobolus brefeldianus* MTCC 5185. Spore suspension from a 3 day old slant was used for preparing the glucose yeast extract seed culture, the composition of which is (grams per liter): yeast extract-3, glucose-10. This was incubated on a rotary shaker at 28° C for 24 hours and was used to initiate the shake flask experiments. Fermentation was carried out in shake flasks in Mikami medium containing (grams per liter): peptone-5; beef extract-5; yeast extract-1.5; glucose-1.5. Chitin at 1% concentration was used as inducer for chitinase production. Medium was adjusted to pH 7. Chitinase activities in the cell free broth after 2 and 3 days were 0.011 IU/ml and 0.016 IU/ml respectively.

**Example 14**

This example illustrates the preparation of **protease** and **chitinase** by submerged fermentation using the strain *Conidiobolus brefeldianus* MTCC 5185. Spore suspension from a 3 day old slant was used for preparing the glucose yeast extract seed culture, the composition of which is (grams per liter): yeast extract-3, glucose-10. This was incubated on a rotary shaker at 28° C for 24 hours and was used to initiate the shake flask experiments. Fermentation was carried out in shake flasks in medium containing (grams per liter): glucose-20; chitin-0.1; di-ammonium hydrogen phosphate-1.6. Medium was adjusted to pH 7 and soyabean meal was added as inducer at 3% concentration. Protease and chitinase activities in the cell free broth after 2 days were 25-29 IU/ml and 0.014-0.016 IU/ml respectively.

**Example 15**

This example illustrates the preparation of **protease** and **laminarinase** by submerged fermentation using the strain *Conidiobolus brefeldianus* MTCC 5185. Spore suspension from a 3 day old slant was used for preparing the glucose yeast extract seed culture, the composition of which is (grams per liter): yeast extract-3, glucose-10. This was incubated on a rotary shaker at 28° C for 24 hours and was used to initiate the shake flask experiments. Fermentation

was carried out in shake flasks in medium containing (grams per liter): glucose-20; di-ammonium hydrogen phosphate-1.6. Medium was adjusted to pH 7 and soyabean meal was added as inducer at 3% concentration. Protease and laminarinase activities in the cell free broth after 2 days were 26-28 IU/ml and 0.95-1.21IU/ml respectively.

**Example 16**

This example illustrates the preparation of **protease** and **keratinase** by submerged fermentation using the strain *Conidiobolus brefeldianus* MTCC 5185. Spore suspension from a 2 day old slant was used for preparing the glucose yeast extract seed culture, the composition of which is (grams per liter): yeast extract-3, glucose-10. This was incubated on a rotary shaker at 28° C for 24 hours and was used to initiate the shake flask experiments. Fermentation was carried out in shake flasks in Mikami medium containing (grams per liter): peptone-5; beef extract-5; yeast extract-1.5; glucose-1.5. Medium was adjusted to pH 7 and chicken feathers (1%) were added separately to each flask as inducer for keratinase. The shake flasks were incubated for 48 h at 28°C on a rotary shaker (200 rpm). Keratinase activity was determined according to Bressollier et al, (Applied Environmental Microbiology, Vol.65 (6), 2570-2576, 1999. One unit of enzyme activity is defined as the amount of enzyme required to cause an increase in absorbance by 0.01 at 595nm per min. Protease activities in the cell free broth after 24 and 48 h were 3.34 IU/ml and 7.90 IU/ml respectively. Keratinase activities for 24 and 48 h were 150.02 and 252.45 U/ml respectively.

**Example 17**

This example illustrates the preparation of the protease and chondroitinase by submerged fermentation using the strain *Conidiobolus brefeldianus* MTCC 5185. Amicon concentrated enzyme sample as described in example 28 showed protease and chondroitinase activities of 222.12 U/ml and 0.1-0.16 U/ml respectively

**Example 18**

This example illustrates the preparation of the protease using various carbon sources by submerged fermentation using the strain *Conidiobolus brefeldianus* MTCC 5185. Spores from a 2 days old MGYP slant were used for preparing the seed culture, the composition of which is (grams per liter) Glucose-10; Yeast extract- 3. This was incubated on a rotary shaker for 24 hours at 28°C and was used to initiate the shake flask experiments. Fermentation was carried out in shake flasks in a medium containing (grams per liter) carbon source-10; Yeast extract- 3, Soyabean meal -30. Fructose, glucose, glycerol, lactose, mannitol and starch at 1% concentration were used as carbon sources. The shake flasks were incubated at 28°C for 72 h on a rotary shaker (200 rpm). The activities for 48 h samples are presented in accompanying Table 2 and forming the part of this specification. Glucose was found to be the best carbon source.

Table 2: Effect of carbon sources on protease production by *Conidiobolus brefeldianus* MTCC 5185

| **Carbon source** | **Relative Activity** (%) |
|---|---|
| **Glucose** | 100.00 |
| **Glycerol** | 27.54 |
| **Starch** | 19.35 |
| **Mannitol** | 33.42 |
| **Lactose** | 44.47 |
| **Fructose** | 54.10 |

**Example 19**

This example illustrates effect of inducers on the preparation of the protease by submerged fermentation using the strain *Conidiobolus brefeldianus* MTCC 5185. Spores from a 3 days old MGYP slant were used for preparing the seed culture, the composition of which is (grams per liter): glucose-10; yeast extract-3. This was incubated on a rotary shaker for 24 hours at 28°C and was used to initiate the shake flask experiments. Fermentation was carried out in shake flasks in a medium containing (grams per liter): glucose-10; yeast extract-3, inducer-20. Following inducers were used: soyabean meal, cotton seed cake, mustard seed cake, groundnut cake, gram flour, mung dal flour, soyatose, skim milk, tryptone, casein and casamino acids. The shake flasks were incubated at 28°C for 72 h on a rotary shaker (200 rpm). The activities for 48 h samples are presented in accompanying Table 3 and forming the part of this specification. All the

inducers tested induced protease production.

Table 3: Effect of inducers on protease production by *Conidiobolus brefeldianus* MTCC 5185

| Inducer | Relative Activity (%) |
|---|---|
| Soyabean meal (2%) | **100.00** |
| Cotton seed cake (2%) | **69.50** |
| Mustard seed cake (2%) | **43.41** |
| Ground nut cake (2%) | **74.64** |
| Gram flour (2%) | **46.76** |
| Mung dal flour (2%)s | **54.07** |
| Soyatose (2%) | **69.97** |
| Skim milk (1%) | **69.75** |
| Tryptone (1%) | **149.77** |
| Casein (1%) | **57.98** |
| Casamino acids (1%) | **143.50** |

**Example 20**

This example illustrates the preparation of the protease using different concentrations of soyabean meal as inducer by submerged fermentation using the strain *Conidiobolus brefeldianus* MTCC 5185. Spores from a 2 days old MGYP slant were used for preparing the seed culture, the composition of which is (grams per liter) Glucose-10; Yeast extract- 3. This was incubated on a rotary shaker for 24 hours at 28°C and was used to initiate the shake flask experiments. Fermentation was carried out in shake flasks in a medium containing (grams per liter): glucose-10; Yeast extract- 3, Soyabean meal -0; 10; 20; 30; 40; 50. The shake flasks were incubated at 28°C for 72 h on a rotary shaker (200 rpm). The activities for 48 h samples are presented in accompanying Table 4 and forming the part of this specification.

Table 4: Effect of soyabean concentration on protease production by *Conidiobolus brefeldianus* MTCC 5185

| **Soyabean meal (%)** | **Relative Activity (%)** |
|---|---|
| **0** | 5.58 |
| **1** | 46.53 |
| **2** | 66.72 |
| **3** | 80.98 |
| **4** | 100.00 |
| **5** | 28.16 |

**Example 21**

This example illustrates protease production in the temperature range of 20 to 45°C by submerged fermentation using the strain *Conidiobolus brefeldianus* MTCC 5185. Spore suspension from a 3 day old slant was used for preparing the MGYP seed culture, the composition of which is (grams per liter): malt extract-3; yeast extract-3; peptone-5 and glucose-10. This was incubated on a rotary shaker at 28° C for 24 hours and was used to initiate the shake flask experiments. Production was carried out in shake flasks in MGYP liquid medium containing (grams per liter): malt extract-3; yeast extract-3; peptone-5 and glucose-10, soyabean meal-20 and adjusted to pH 6.5-7. The flasks were incubated at temperatures ranging from 20 to 45°C on a rotary shaker (200 rpm) for 48 h. Protease production was observed in the temperature range of 20 to 37°C with highest activities at 28°C (Table 5).

Table 5: Effect of Temperature on protease production by *Conidiobolus brefeldianus* MTCC 5185

| **Temperature (°C)** | **Relative Activity (%)** |
|---|---|
| **20** | 82.42 |
| **28** | 100 |
| **37** | 20.31 |
| **45** | 0.87 |

**Example 22**

This example illustrates the preparation of the protease by submerged fermentation in the pH range 5 to 10 using the strain *Conidiobolus brefeldianus* MTCC 5185. Spore suspension from a 3 day old slant was used for preparing the GYE seed culture, the composition of which is (grams per liter): yeast extract-3 and glucose-10. This was incubated on a rotary shaker at 28° C for 24 hours and was used to initiate the shake flask experiments. Fermentation was carried out in shake flasks in a medium containing (grams per liter): glucose-20; yeast extract-3, soyabean meal-20. Medium pH was varied from pH 5 to 10 by addition of sterile NaOH or HCl. The shake flasks were incubated at 28°C on a rotary shaker (200 rpm) for 48 h. Protease production was observed in the pH range 5 to 10 (Table 6).

Table 6: Effect of pH on protease production by *Conidiobolus brefeldianus* MTCC 5185

| **pH** | **Relative Activity (%)** |
|---|---|
| **5.00** | 98.91 |
| **5.45** | 100.00 |
| **6.12** | 96.75 |
| **7.59** | 90.26 |
| **8.40** | 87.39 |
| 9.00 | 74.17 |
| **9.50** | 45.50 |
| **10.05** | 29.00 |

**Example 23**

This example illustrates the pH range in which the protease secreted by the said strain is active. For determination of optimum pH for protease, the enzyme was diluted and assayed in buffers of different pH ranging from 5 to 12. Following buffers at 0.1M concentration were used: Acetate (pH 5), sodium phosphate buffer (pH 6, 7), Tris HCl buffer (pH 8) and carbonate bicarbonate (pH 9 and 10), sodium phosphate-NaOH (pH 11, 12) and KCl-NaOH (pH 12.0). The results are presented in accompanying Table 7 and forming the part of this specification. It is observed that the enzyme is active between pH 6 and 11.

Table 7: Optimum pH for Protease

| pH | Relative Activity (%) |
|---|---|
| 5.10 | 3.04 |
| 5.94 | 15.88 |
| 7.03 | 55.83 |
| 8.11 | 63.95 |
| 9.01 | 100.00 |
| 10.11 | 61.66 |

(continued)

| pH | Relative Activity (%) |
|---|---|
| 11.00 | 30.37 |
| 11.97 | 3.52 |
| 12.15 | 1.09 |

**Example 24**

This example illustrates the temperature range in which the protease secreted by the said strain is active. For determination of optimum temperature, protease activity was determined with carbonate bicarbonate buffer (0.1M, pH 9) at temperatures ranging from 30 to 70°C. The results of the experiment have been illustrated in Table 8 accompanying and forming the part of this specification. It is observed that the enzyme is active between 30 to 60°C.

Table 8-: Optimum Temperature of Protease

| Temperature(°C) | Residual Activity (%) |
|---|---|
| 30 | 17.26 |
| 40 | 66.71 |
| 50 | 100.00 |
| 60 | 13.39 |
| 70 | 4.81 |

**Example 25**

Stability of the said protease at 40°C with respect to time was determined by incubating the crude protease in phosphate buffer pH 7.0 at 40 °C up to 2 h. The crude protease was produced as described in example 4 .Aliquots were removed at regular intervals of 15 min and residual activity was estimated at 50°C, pH 9. The results are illustrated in Table 9 wherein the protease was stable up to 2 h at 40°C and retained approximately 60% activity.

Table 9: Temperature Stability of Protease at 40°C

| Time (min) | Residual Activity (%) |
|---|---|
| 0 | 100.00 |
| 15 | 99.24 |
| 30 | 97.96 |
| 45 | 94.54 |
| 60 | 88.91 |
| 75 | 81.53 |
| 90 | 76.14 |
| 105 | 67.27 |
| 120 | 59.54 |

**Example 26**

This example illustrates the concentration of protease by lyophilization. The protease (100 ml) produced as described in example 4 and having 36.33 U/ml activity was lyophilized for 6 hours (-55°C) till liquid completely evaporated leaving dry hygroscopic powder. The lyophilized powder was dissolved in deionozed water and protease activity was estimated. Lyophilized sample showed an activity of 191.88 U/ml. Recovery of protease was found to be above 95%.

This indicates that the protease stable to lyophilization.

**Example 27**

This example illustrates the concentration of protease by ultrafiltration. Protease produced as described in example 4 was centrifuged at 10,000 rpm. The clear supernatant (1200 ml) having activity of 25.84 IU/ml was concentrated in Amicon membrane filtration unit on YM-3 membrane with a molecular weight cut off of 3,000 daltons. Volume after concentration was 150 ml with 206.72 IU/ml protease activity. The recovery after filtration was 95-98%. In another set 1000 ml of supernatant was also concentrated on PM-10 membrane (10,000 daltons cut off) to 100 ml with 85% recovery.

**Example 28**

This example illustrates the concentration of protease by ammonium sulphate precipitation with 90% saturation. Protease produced as described in example 6 was centrifuged at 10,000 rpm. The clear supernatant (1000 ml) having activity of 31.29 IU/ml was used for concentration. Precipitation was carried out at 4°C by adding 600 g of ammonium sulphate with slow continuous mixing. Stirring was continued for another 2 h for complete precipitation and allowed to settle in cold. The clear supernatant was decanted and the volume of the suspension was reduced to 250 ml resulting in 4 fold concentration of protease. The protease activity in the suspension was estimated after dissolving the precipitate obtained after centrifugation at 10, 000 rpm in the 10 mM Tris HCl buffer, pH 8. There was more than 90% recovery of protease and the activity in the suspension was 118.12 IU/ml.

**Example 29**

This example illustrates the stability of protease from *Conidiobolus brefeldianus* MTCC 5185 in presence of detergents. The crude protease produced as described in example 4 was incubated with 0.7 mg/ml detergents (final concentration) at 40°C up to 1h. Samples were removed at intervals of 15 min and residual activity was measured and expressed as percentage of initial activity with respective detergents taken as 100%. The protease was stable in the presence of all detergents and retained 75-94% activity after 15 minutes depending on the detergent (Table 10). Even after 1 h, around 35-50% activity was retained.

Table 10: Stability of protease in presence of detergents

| Detergent (0.7mg/ml) | Residual Activity (%) | | | | |
|---|---|---|---|---|---|
| | 0 min | 15 min | 30 min | 45min | 60min |
| Nil | 100.00 | 94.28 | 91.91 | 74.89 | 71.94 |
| Arial | 100.00 | 78.28 | 42.04 | 42.69 | 35.47 |
| Rin | 100.00 | 75.06 | 53.34 | 50.72 | 39.82 |
| Surf Excel | 100.00 | 79.45 | 66.89 | 52.17 | 43.89 |
| Tide | 100.00 | 92.04 | 57.86 | 52.61 | 50.21 |

**Example 30**

This example illustrates that the protease secreted by *Conidiobolus brefeldianus* MTCC 5185 is active in presence of various metals. The crude protease produced as described in example 4 was used. Protease activity was estimated in presence of metals. Stock solutions of metals (100mM) were prepared and added to the assay mixture at final concentration of 5mM. The results of the experiment have been illustrated in Table 11 accompanying and forming the part of this specification. Protease was active in presence of Ca, Cd, Co, K, Mg and Mn while Ni and Zn resulted in 35-40% inhibition. Cu and Hg totally inhibited the protease activity.

Table 11: Effect of metals on protease activity

| **Metal (5mM)** | **Relative Activity (%)** |
|---|---|
| Nil | 100 |
| $AgNo_3$ | - |

(continued)

| Metal (5mM) | Relative Activity (%) |
|---|---|
| $CaCl_2$ | 118.25 |
| $CdCl_2$ | 114.30 |
| $CoCl_2$ | 118.71 |
| $CuCl_2$ | 12.78 |
| $CuSO_4$ | - |
| $HgCl_2$ | 3.27 |
| KCl | 158.50 |
| $MgCl_2$ | 132.24 |
| $MgSO_4$ | 135.87 |
| $MnCl_2$ | 104.49 |
| $MnSO_4$ | 108.39 |
| $NiCl_2$ | 65.60 |
| $ZnCl_2$ | 60.27 |
| $ZnSO_4$ | 58.03 |

**Example 31**

This example illustrates the temperature range in which the protease secreted by the said strain is stable for 1h. For determining the temperature stability, crude ultrafiltered protease as described in example 27 was incubated at temperatures ranging from 4 to 70°C for 1h and the residual activity was measured at 50°C, pH 9. The results are illustrated in Table 12 wherein it is observed that the enzyme is stable up to 50°C.

Table 12: Temperature Stability of Protease

| Temperature(°C) | Residual Activity (%) |
|---|---|
| 4 | 100.00 |
| 30 | 96.54 |
| 40 | 89.55 |
| 50 | 43.81 |
| 60 | 0.84 |
| 70 | 0.45 |

**Example 32**

This example illustrates the stability of protease at 50°C. Stability of three different samples of the said protease at 50°C with respect to time was determined by incubating the protease at 50 °C up to 2 h. The crude culture filtrate, ammonium sulphate precipitate and ultrafiltered (membrane) protease samples as described in examples 4, 27 and 28 were used for stability studies. Aliquots were removed at regular intervals of 30 min and residual activity was estimated at 50°C, pH 9. The results are illustrated in Table 13 wherein all the protease samples retained more than 60% activity at 50°C after 30 min. Ammonium sulphate precipitated sample was found to be more stable which retained around 40% activity after 2 h while culture filtrate and PM-10 Retentate retained around 25-27% activity after 2 h.

Table 13: Temperature Stability of Protease at 50°C

| Time (min) | Crude culture filtrate | Ammonium sulphate precipitate | PM-10 Retentate |
|---|---|---|---|
| **0** | 100.00 | 100.00 | 100.00 |

(continued)

| Time (min) | Crude culture filtrate | Ammonium sulphate precipitate | PM-10 Retentate |
|---|---|---|---|
| **30** | 62.33 | 67.80 | 60.30 |
| **60** | 44.84 | 55.54 | 44.01 |
| **90** | 33.30 | 51.17 | 32.51 |
| **120** | 25.56 | 40.86 | 27.23 |

**Example 33**

This example illustrates the stability of the protease in presence of sugar alcohols. Ultrafiltered protease as described in example 27 was incubated at 50°C in presence of glycerol, mannitol, sorbitol and xylitol at 20% concentration up to 3 h. Aliquots were removed at regular intervals of 30 min and residual activity was estimated at 50°C, pH 9. The results are illustrated in Table 14. All the sugar alcohols increased the stability of enzyme and more than 30% activity was retained after 3 h. Among them, sorbitol offered greater thermostability to the protease with 47% residual activity.

Table 14: Temperature Stability of Protease at 50°C in presence of sugar alcohols

| Time (Min) | Control | glycerol | Mannitol | sorbitol | xylitol |
|---|---|---|---|---|---|
| 0 | 100 | 100 | 100 | 100 | 100 |
| 30 | 69.71 | 74.68 | 74.06 | 84.01 | 76.31 |
| 60 | 40.58 | 62.01 | 64.42 | 70.66 | 63.36 |
| 90 | 31.92 | 49.08 | 58.13 | 62.31 | 52.42 |
| 120 | 26.79 | 36.97 | 48.66 | 51.57 | 44.61 |
| 180 | 19.00 | 31.73 | 40.71 | 47.11 | 39.25 |

**Example 34**

This example illustrates the stability of the protease in presence of sorbitol and trehalose. Ultrafiltered protease as described in example 27 was incubated at 50°C in presence of 20, 40 and 50% sorbitol and trehalose up to 4 h. Aliquots were removed at regular intervals of 60 min and residual activity was estimated at 50°C, pH 9. The results are illustrated in Table 15. Stability increased with increasing concentration of trehalose and sorbitol. There was nearly 5 to 6 fold increase in thermostability with 50% sorbitol and trehalose which retained nearly 80% activity even after 4 h while control without additives showed 12.67% residual activity.

Table 15: Temperature Stability of Protease at 50°C in presence of Trehalose and Sorbitol

| | | **Trehalose (%)** | | | **Sorbitol (%)** | | |
|---|---|---|---|---|---|---|---|
| **Time (h)** | **Control** | **20** | **40** | **50** | **20** | **40** | **50** |
| **0** | 100.00 | 100.00 | 100.00 | 100.00 | 100.0 | 100.0 | 100.0 |
| **1** | 43.81 | 78.14 | 85.27 | 92.32 | 77.28 | 87.00 | 91.31 |
| **2** | 23.34 | 62.15 | 75.99 | 89.98 | 49.25 | 70.69 | 88.44 |
| **3** | 16.11 | 46.12 | 75.47 | 85.89 | 42.99 | 61.54 | 79.44 |
| **4** | 12.67 | 39.80 | 71.11 | 81.89 | 33.11 | 58.57 | 78.25 |

**Example 35**

This example illustrates the stability of the protease in presence of salts. Ultrafiltered protease as described in example 27 was incubated at 50°C in presence of 10mM $CaCl_2$, 0.5M NaCl, 0.5M glycine and 15 % Ammonium sulphate up to 3 h. Aliquots were removed at regular intervals of 30 min and residual activity was estimated at 50°C, pH 9. The

results are illustrated in Table 16. Stability increased in presence of all the salts. Calcium chloride and ammonium sulphate offered greater protection to thermal denaturation where the residual activity was around 55% after 3 h while control showed around 20% residual activity.

Table 16: Temperature Stability of Protease at 50°C in presence of salts

| **Time (min)** | **Residual Activity (%)** | | | | |
|---|---|---|---|---|---|
| | **Control** | **$CaCl_2$ 10mM** | **0.5M NaCl** | **0.5M glycine** | **15% $(NH_4)_2SO_4$** |
| **0** | 100.00 | 100.00 | 100.00 | 100.00 | 100.00 |
| **30** | 73.37 | 91.70 | 77.79 | 74.85 | 82.27 |
| **60** | 48.40 | 83.17 | 54.11 | 54.19 | 71.87 |
| **90** | 37.78 | 70.83 | 49.26 | 43.16 | 64.72 |
| **120** | 20.76 | 63.83 | 48.83 | 39.89 | 60.25 |
| **180** | 19.39 | 54.65 | 37.44 | 31.62 | 55.19 |

**Example 36**

This example illustrates the effect of water miscible as well as water immiscible organic solvents on stability of crude protease at 28°C. Following organic solvents were used: acetone, acetonitrile, 1-butanol, dimethylsulphoxide, isopropanol and methanol. Crude protease produced as described in example 4 was incubated at 28°C with 20% (v/v) organic solvents (effective concentration). Samples were removed at different time intervals and residual activity was estimated. Sample without organic solvent served as control. Initial activity with respective solvents was taken as 100% (Table 17). The protease was stable in presence of organic solvents with the exception of butanol which showed 75.26% residual activity while more than 95% activity was retained in all other solvents up to 5 h. After 12 h incubation at 28°C, except butanol, protease retained more than 50% activity while the residual activity in butanol was around 40%. (Table 17).

Table 17: Stability of protease in presence of organic solvents at 28°C

| Type of the solvent | Solvent | | | |
|---|---|---|---|---|
| | | 0 h | 5 h | 12 h |
| Control | None | 100 | 97.70 | 76.65 |
| Water miscible | Acetone | 100 | 95.66 | 55.51 |
| | Acetonitrile | 100 | 98.31 | 49.90 |
| | Isopropanol | 100 | 100.29 | 57.79 |
| | Methanol | 100 | 100.13 | 52.64 |
| Water immiscible | Butanol | 100 | 75.26 | 39.93 |
| | DMSO | 100 | 98.12 | 54.55 |

**Example 37**

This example illustrates the effect of water miscible as well as water immiscible organic solvents on stability of crude protease at 37°C. Following organic solvents were used: acetone, acetonitrile, 1-butanol, dimethylsulphoxide, isopropanol and methanol. Crude protease produced as described in example 4 was incubated at 37°C, pH 7 with 20% (v/v) organic solvents (effective concentration). Samples were removed at different time intervals and residual activity was estimated. Sample without organic solvent served as control. Initial activity with respective solvents was taken as 100% (Table 18). The protease was stable in presence of organic solvents with the exception of acetonitrile, butanol and isopropanol which showed less than 35% residual activity while more than 50% activity was retained in acetone, methanol and dimethylsulphoxide after 2h (Table 18).

Table 18: Stability of protease in presence of organic solvents at 37°C

| Type of the solvent | Solvent | Residual Activity (%) | | | | |
|---|---|---|---|---|---|---|
| | | 0 | 30 min | 60 min | 120 min | 240 min |
| Control | None | 100 | 96.40 | 93.51 | 85.85 | 79.04 |
| Water miscible | Acetone | 100 | 91.66 | 81.10 | 60.57 | 29.53 |
| | Acetonitrile | 100 | 37.19 | 36.40 | 17.52 | 13.45 |
| | Isopropanol | 100 | 46.06 | 34.35 | 18.73 | 17.11 |
| | Methanol | 100 | 59.43 | 59.45 | 48.36 | 16.60 |
| Water immiscible | Butanol | 100 | 47.57 | 30.60 | 33.00 | 13.53 |
| | DMSO | 100 | 69.48 | 68.94 | 48.70 | 28.53 |

**Example 38**

This example illustrates the non-collagenase nature of the protease from *Conidiobolus brefeldianus* MTCC 5185 by fluorescence studies using NMITLI-1 and Collagenase-1 substrates. Initially fluorescence experiments were done with crude as well as purified proteases against NMITLI-1 substrate. Fluorescence experiments were carried out in triethanolamine buffer (TEA) pH 8 at room temperature. Typically, the assay contained 290μl of 100mM TEA buffer pH 8, having NMITLI-1 substrate concentration of 1.8μM (10μl of 112μM stock solution). The reaction was initiated by adding 5μl of enzyme samples. The excitation wavelength was 340nm and the emission was scanned from 425-625nm, at different intervals of time. There was three fold increase in the fluorescence of AEDANS chromophore (+++) indicating proteolytic nature of the enzyme samples.

**NMITLI substrate -I**

**Dabcyl-Gaba-Arg-Pro-Leu-Gly-Ala-Ala-Ala-Lys-Val-Gaba-Cys-Lys-$NH_2$**

**AEDANS**

As crude as well as purified protease samples showed activity against NMITLI-1 substrate, they were also screened for collagenase activity with Collagenase substrate-I. Fluorescence experiments were carried out in triethanolamine buffer (TEA) pH 8 at room temperature. Typically, the assay contained 290μl of 100mM TEA buffer pH 8, having substrate (Collagenase substrate-I) concentration of 1.6μM (5μl of 80mM stock solution). The reaction was initiated by adding 5μl of enzyme samples. The excitation wavelength was 340nm and the emission was scanned from 425-625nm, at different intervals of time. There is no increase in the fluorescence of AEDANS chromophore (-) indicating absence of collagenase activity.

**Collagenase substrate - I**

**Dabcyl-Gaba-Lys-Gly-Gly-Pro-Leu-Gly-Pro-Pro-Gly-Pro-Gly-Gly-Cys-Lys-$NH_2$**

**AEDANS**

These results indicate that the crude as well as purified proteases from *Conidiobolus brefeldianus* MTCC 5185 were inactive against Collagenase substrate-1 showing non-collagenase nature of the proteases.

**Example 39**

This example illustrates the determination of activity of the protease from *Conidiobolus brefeldianus* MTCC 5185 towards elastin-orcin. The reaction mixture contained an aliquot of suitably diluted protease enzyme and 20mg elastin-orcin in 50 mM Tris HCl buffer pH 8.0 in a total volume of 3ml. Heat inactivated enzyme (by boiling for 15 min) was taken as blank. After incubation at 50°C for 30 min, contents were filtered on Whatman No 1 filter paper and absorbance of the filtrate was measured at 578nm. One unit of enzyme activity is defined as the amount of enzyme required to cause an increase in absorbance by one unit at 578nm in one minute. The ammonium sulphate precipitated suspension as described in example 28 having caseinolytic activity of 118.12 IU/ml showed activity of 3.84 U/ml against elastin-orcin.

**Example 40**

This example illustrates the determination of azocoll activity of the protease from *Conidiobolus brefeldianus* MTCC 5185. The reaction mixture contained an aliquot of suitably diluted protease and 10mg azocoll in 0.05M Tris HCl buffer pH 8.0 in a total volume of 2.5ml. Heat inactivated enzyme (by boiling for 15 min) was taken as blank. After incubation at 37°C for 10min, the reaction was terminated by filtering through Whatman No.1, filter paper. The absorbance of filtrate was measured at 580nm. One unit of enzyme activity is defined as the amount of enzyme required to cause an increase in absorbance by one unit at 580nm per minute. The crude culture filtrate grown as described in example 3 had an azocoll activity of 16-18 U/ml.

**Example 41**

This example illustrates the determination of azocasein activity of the protease from *Conidiobolus brefeldianus* MTCC 5185. The reaction mixture contained an aliquot of suitably diluted protease and 1mg azocasein in 0.05 M sodium carbonate buffer pH 9.0 in a total volume of 500μl. Heat inactivated enzyme (by boiling for 15 min) was taken as blank. After incubation at 50°C for 30 min, the reaction was terminated by addition of 500μl of 10% TCA. After cooling on ice for 15 min, contents were centrifuged at 8000rpm for 10 min. To 800μl of supernatant, 200μl of 1.8M NaOH was added and absorbance was measured at 420nm. One unit of enzyme activity is defined as the amount of enzyme required to cause an increase in absorbance by one unit at 420nm per minute. The crude culture filtrate grown as described in example 3 showed an activity of 40-45 U/ml.

**Example 42**

This example illustrates the activity of crude protease from *Conidiobolus brefeldianus* MTCC 5185 towards N-α-benzoyl-DL-arginine-p-nitroanilide (BAPNA). The assay mixture consisting of 0.16 ml of 3 mM N-α-benzoyl-DL-arginine-p-nitroanilide (BAPNA) in DMSO, 0.5 ml of 50 mM phosphate buffer pH 7.2 and 0.1 ml of appropriately diluted enzyme sample was incubated at 37°C for 1 h. Reaction was terminated by addition of 0.5 ml of 1 M $Na_2CO_3$. Absorbance was measured at 420 nm. One unit of activity is defined as the amount of enzyme required to cause an increase in absorbance by one unit at 410nm in one minute. The crude enzyme as described in example 3 having caseinolytic activity of 20.7 U/ml showed an activity of 1.41 U/ml

**Example 43**

This example illustrates the determination of protease activity from *Conidiobolus brefeldianus* MTCC 5185 with casein, hemoglobin and bovine serum albumin as substrates. The crude culture filtrate grown as described in example 3 was concentrated by ultra-filtration on PM-10 membrane and used for the studies. The reaction mixture contained an aliquot of suitably diluted protease enzyme and 10mg substrate in 0.1M sodium carbonate buffer pH 9.0 in a total volume of 2ml. After incubation at 50°C for 10min, the reaction was terminated by the addition of 3ml of 5% trichloroacetic acid (acidified with concentrated hydrochloric acid). The precipitate formed was filtered through Whatman No.1 filter paper after standing at room temperature for 30min. The absorbance of trichloroacetic acid soluble fraction was measured at 280nm. The protease is able to degrade the above substrates to varying degrees (Table 19).

Table 19: Activity towards substrates

| Substrate | Relative Activity (%) |
|---|---|
| Casein | 100.0 |

(continued)

| Substrate | Relative Activity (%) |
|---|---|
| Haemoglobin | 82.31 |
| Bovine Serum Albumin | 53.71 |

**Example 44**

This example illustrates the effect of inhibitors on protease activity of *Conidiobolus brefeldianus* MTCC 5185. Following inhibitors were studied: phenylmethylsulfonyl fluoride (PMSF), ethylene-diaminetetraacetic acid (EDTA), iodoacetic acid, and dimeth ylsulphoxide (DMSO). The crude culture filtrate grown as described in example 3 was concentrated by ultra-filtration on PM-10 and YM-3 membranes as described in example 27 and used for inhibition studies. Both the protease preparations were pre-incubated in 100mM Tris-HCl buffer (pH 8.0) with each inhibitor for 1 h and the residual protease activity was measured at 50°C, pH 9.0. Protease without inhibitor served as control. Protease was completely inhibited by 1mM PMSF indicating it to be a serine protease (Table 20).

Table 20: Effect of inhibitors

| Inhibitor | Inhibition (%) | |
|---|---|---|
| | YM-3 Retentate | PM -10 Retentate |
| Control | 0.0 | 0.0 |
| 20mM EDTA | 3.93 | 0.0 |
| 10 mM Iodoacetate | 16.54 | 0.0 |
| 1 mM PMSF (in DMSO) | 90.24 | 79.27 |
| DMSO | 1.37 | 3.02 |

**Example 45**

This example illustrates the effect of inhibitors on protease activity of *Conidiobolus brefeldianus* MTCC 5185. Following inhibitors were studied: N-tosyl-L-phenylalanine chloromethyl ketone (TPCK), N-tosyl-L-lysine chloromethyl ketone (TLCK), and Benzamidine. Crude protease was pre-incubated with each inhibitor in 100mM Tris-HCl buffer (pH 8.0) for 30 min and the residual protease activity was measured 50°C, pH 9.0. Protease without inhibitor served as control. Inhibition by TPCK and TLCK was 9 and 29% respectively while Benzamidine did not inhibit indicating that the protease does not have trypsin like activity (Table 21).

Table 21: Effect of inhibitors

| Inhibitor | Concentration | Inhibition (%) |
|---|---|---|
| Control | - | 0 |
| TPCK | 5mM | 9.02 |
| TLCK | 5mM | 29.22 |
| Benzamidine | 5mM | 0 |

**Example 46**

This example illustrates the pH range in which the lipase secreted by the said strain is active. For determination of optimum pH for lipase, the enzyme was assayed at 50°C and pH ranging from 4 to 9 by titrimetric method. Following buffers were used: acetate (pH 4 and 5), phosphate (pH 6, 7), Tris HCl (pH 8) and carbonate bicarbonate (pH 9). The results are presented in accompanying **Table 22** and forming the part of this specification. It is observed that the enzyme is active between pH 4 and 9.

Table 22: Optimum pH of lipase from *Conidiobolus brefeldianus* MTCC 5185

| pH | Relative Activity (%) |
|---|---|
| 4 | 11.08 |
| 5 | 15.27 |
| 6 | 60.00 |
| 7 | 100.00 |
| 8 | 43.08 |
| 9 | 29.17 |

**Example 47**

This example illustrates the temperature range in which the lipase secreted by the said strain is active. For determination of optimum temperature, lipase activity was determined by titrimetric method at pH 7 and at temperatures ranging from 30 to 60°C. The results of the experiment have been illustrated in Table 23 accompanying and forming the part of this specification. It is observed that the enzyme is active between 30 to 60°C.

Table 23: Optimum Temperature of lipase from *Conidiobolus brefeldianus* MTCC 5185

| Temperature (°C) | Relative Activity (%) |
|---|---|
| 30 | 18.70 |
| 40 | 58.54 |
| 50 | 100.00 |
| 60 | 56.91 |

**Example 48**

This example illustrates the purification of the *Conidiobolus brefeldianus* MTCC 5185 protease to homogeneity. The fungus was grown as described in example 4 and the clear supernatant obtained after centrifugation at 10,000rpm for 10min was used as the source of enzyme. Fractional ammonium sulphate precipitation of the broth was carried out at 4°C and dialyzed ammonium sulphate precipitate (50-80% saturation) was loaded on a diethyl aminoethyl cellulose (DEAE-cellulose) column (2.5 cm x 25 cm) equilibrated with 50mM phosphate buffer pH 7.0. The un-adsorbed enzyme was eluted with 50mM phosphate buffer pH 7.0 at a flow rate of 20ml/h. Fractions showing protease activity were pooled, concentrated by speed-vacc and loaded on Sephadex-G-100 column (1.2 cm X 150cm) equilibrated with 50 mM phosphate buffer, pH 7. Column was eluted with 50mM phosphate buffer pH 7.0 at a flow rate of 12ml/h. Fractions showing protease activity were pooled and stored at -20°C.

**Example 49**

This example illustrates the purity and determination of molecular weight of purified protease by sodium dodecylsulphate polyacryalmide gel electrophoresis (SDS-PAGE) and matrix-assisted laser desorption ionization time-of-flight (MALD-TOF). The molecular mass marker kit for SDS-PAGE from M/s BioRad (Cat No. 161-0305) consisted of carbonic anhydrase (35.88 kDa), soyabean trypsin inhibitor (27.86 kDa), lysozyme (18.81 kDa), bovine serum albumin (87.55 kDa), phosphorylase b (101.47 kDa) and ovalbumin (52.74 kDa). The molecular mass of the enzyme by SDS-PAGE was found to be around 29 kDa which was slightly lower than the molecular weight of carbonic anhydrase (Fig. 6).

The molecular mass of purified protease was also determined by using MALDI-TOF mass spectrometry using a Voyager DE-STR (Applied Biosystems) equipped with a 37-nm nitrogen laser. The purified enzyme was mixed with equal volume of sinapinic acid in acetonitrile and 20μl of prepared sample was placed on MALDI plate for analysis. The purified enzyme showed the molecular weight of 27.8 kDa in MALDI-TOF (Fig.7) which was nearly similar to the molecular weight of 29 kDa obtained by SDS-PAGE

**Example 50**

Crude protease produced as described in example 6 was spray dried with 10% and 15% maltodextrin as additives with inlet temperatures of 150-160°C and outlet temperatures of 60-65°C and 70-80°C respectively with 70-80% recoveries. The stability of spray dried protease was studied at temperatures ranging from 4°C to 37°C. Samples were distributed in vials and stored at 4°C, 28°C and 37°C. One vial incubated at each temperature was removed at regular intervals and residual activity was checked. Spray dried protease was stable up to 25months (760 days) at all the temperatures tested and retained 85-90% activity (Fig. 8a and 8b).

**Example 51**

This example illustrates the removal of silver from waste X-ray film using crude protease, biomass filtrate and biomass of *Conidiobolus brefeldianus* MTCC 5185. 5 g black X-ray film cut in to 1 cm x 1 cm pieces were washed with distilled water. These pieces were incubated with 20 U of protease in 10 mM carbonate bicarbonate buffer, pH 9 in a total volume of 50ml in 250 ml conical flask at 37°C and 180 rpm. A control without protease was also incubated under identical conditions. Stripping of gelatin layer from the film started within few minutes and was complete within 1 h. The solution looked blackish due to the blackish silver and the film was clean and white (Fig. 9a). For removal using biomass filtrate, the organism was grown on MGYP liquid medium the composition of which is (grams per liter): malt extract-3; yeast extract-3; peptone-5 and glucose-10 for 24 h with shaking at 200 rpm and 28°C. Four grams of biomass (wet weight) was collected by centrifugation at 10,000rpm, washed twice with 50 ml of double distilled water and suspended in 50 ml distilled water and incubated at 28°C, 180 rpm. After 2 h, filtrate was collected by filtration on Whatman No 1 filter paper. Two grams of washed X-ray film (cut in to 2 cm x 2 cm pieces) was incubated with 50 ml of biomass filtrate at 37°C with shaking at 200 rpm. A control without biomass filtrate was incubated under identical conditions. Stripping of gelatin layer from the film started within few minutes and was complete within 1 h. The solution looked blackish due to the blackish silver and the film was clean and white (Fig. 9b). For removal using biomass, the fungus was grown aerobically in MGYP liquid medium as described above at 28°C, 200 rpm for 24 h. The biomass was harvested by centrifugation and washed twice with double distilled water. Wet biomass (0.1 g) was applied as a paste on washed X-ray film (3 cm x 2 cm) and incubated at 37°C. Complete removal of gelatin layer and appearance of clean film was observed after 1h. (Fig. 9c).

**Example 52**

This example illustrates the use of protease from *Conidiobolus brefeldianus* MTCC 5185 for degumming of silk. Silk twists were dried in oven at 110°C for 3 h (or till constant weight reached). 1 g dried silk was incubated with crude protease prepared as described in example 3 at 50°C with intermittent manual shaking for 1h. Four different protease concentrations (50 to 200 U/g of silk) were used for degumming. Solid to liquid ratio was kept at 1:30. After degumming, silk fiber was washed first with tap water (cold wash) followed by a hot wash (65°C for 20 min). After washing, silk was air dried overnight before drying in oven (110-120°C) for 2 to 3 h (or till constant weight reached) and weight of the degummed silk was recorded. Loss in weight after degumming was calculated from the difference in initial and final weights. Loss in weight after degumming increased with increase in enzyme concentration and ranged from 13 to 22% (Table 24). The weight loss for conventionally degummed fiber was around 24.4%. The fiber obtained had smooth feel and luster (Fig. 10 a&b). The scanning electron micrographs showed the removal of serecin without affecting the strength properties of the fiber (Fig. 11a & b). The insoluble residue in the degumming liquor (due to serecin removal) increased with increase in protease concentration (Fig. 12).

Table 24: Loss in weight after degumming

| Protease (U/g) | Loss in weight (%) |
|---|---|
| 50 | 13.49 |
| 100 | 17.485 |
| 150 | 18.275 |
| 200 | 22.619 |

**Example 53**

This example illustrates the use of protease from *Conidiobolus brefeldianus* MTCC 5185 for degumming of silk and separation of serecin proteins/peptides from degumming liquor. 4 g silk twists was dried in oven for 3 h at 110°C (or till constant weight reached) and incubated with 800 U of protease prepared as described in example 3 at 50°C with intermittent manual shaking for 1h. Solid to liquid ratio was kept at 1:30. After degumming, silk fiber was washed first with tap water (cold wash) followed by a hot wash (65°C for 20 min). After washing, silk was air dried overnight before drying in oven (110-120°C) for 2 to 3 h (or till constant weight reached) and weight of the degummed silk was recorded. Loss in weight after degumming was around 20%. The insoluble sericin (whitish residue) from degumming liquor (DGL) was separated by centrifugation at 6000 rpm for 5 min. This insoluble residue was suspended in double distilled water and was subjected to sodium dodecyl sulphate polyacrylamide gel electrophoresis (SDS-PAGE). Serecin obtained by conventional method (autoclaving at 121 for 60 min) was included for comparison. Three protein bands were visualized after silver staining incase of enzymatic degumming whereas there was a streak on the gel for serecin obtained by conventional method, indicating its complete degradation (Fig. 13).

**Example 54**

This example illustrates the process for unhairing of goat skins by paste method using crude protease from *Conidiobolus brefeldianus* MTCC 5185. The protease produced as described in example 6 was subjected to ammonium sulphate precipitation (90% saturation) and used for dehairing studies. Wet salted goat skins were taken and soaked for 6 hours. After soaking the weight of the skin was noted. A paste of 0.5 to 1.5% protease and 10% water based on the soaked weight was applied on the flesh side of the skin and piled for 6 hours. A corresponding chemical based control process was also carried out using paste of 10% lime, 3% sulfide and 15% water. The control skins were also piled for 6 hours. Then both control and experimental skins were unhaired and the unhairing efficacy of experimental skins ranged from 85 to 100% which was found to be similar to the unhairing efficacy in chemical based unhairing process. The pelts were clean and free from scud. The dehaired pelts were tanned and further processed into crust leathers and the quality of the crust leathers was also assessed. The quality of leather in terms of tensile strength, tear strength and grain bursting strength were comparable to that obtained by lime and sulphide based dehairing. The reduction in pollution load was also assessed by analyzing the waste streams of both control and experimental process. The results indicate that there was significant reduction in COD and sulfide. The BOD/COD ratio of the waste stream of experimental process was found to be 0.72 which indicates that the degradability and treatability of the wastewater is much better compared to the wastewater of chemical process system, the BOD/COD ratio of which was 0.4.

**Example 55**

This example illustrates the process for unhairing of cow hides using crude protease from *Conidiobolus brefeldianus* MTCC 5185. The protease produced as described in example 6 was subjected to ammonium sulphate precipitation (90% saturation) and used for dehairing studies. Wet salted cow hides were washed and soaked for 8 hours. After soaking, the weight of the hides was noted. The hides were treated in drums using 15% water and 3-4 % enzyme. A corresponding chemical based control process was also carried out using 150% water, 10% lime and 3% sodium sulfide. In the case experimental process, the drum was run intermittently for 10 minutes for every hour. After six hours of running, the pelts were washed. The unhairing was 100%. In the case of control, the drum was run for a day intermittently for 5 minutes for every hour. Next day the pelts were unhaired and the unhairing efficacy was 100%. The experimental pelts were clean and free from scud. The dehaired pelts were tanned and further processed into crust leathers and the quality of the crust leathers was also assessed. The quality of leather in terms of tensile strength, tear strength and grain bursting strength were comparable to that obtained by lime and sulphide method of dehairing. The reduction in pollution load was also assessed by analyzing the waste streams of both control and experimental process. The results indicate significant reduction in COD and sulfide with BOD/COD ratio of 0.69 in the case of experiment, which indicates that the degradability and treatability of the wastewater are enhanced.

**Example 56**

This example illustrates the process for unhairing of sheep skins by paste method using crude protease from *Conidiobolus brefeldianus* MTCC 5185. The protease was produced as described in example 6. This was subjected to ammonium sulphate precipitation (90% saturation) and used for dehairing studies of sheep skins. Wet salted sheep skins were washed and soaked for 5 hours. After soaking the weight of the skin was noted. A paste of 0.5 to 1.5% protease and 10% to 15% of water based on the soaked weight was prepared and applied on the flesh side of the skin. The skins were piled for 6 hours. A control process based on chemicals was also carried out using paste of 10% lime,

3% sulfide and 15% water. The control skins were also piled for 6 hours. Then both control and experimental skins were unhaired and the unhairing efficacy of experimental skins ranged from 85 to 100% which was found to be similar to the unhairing efficacy in chemical based unhairing process. The pelts were clean and free from scud. The dehaired pelts were tanned and further processed into crust leathers and the quality of the crust leathers was also assessed. The quality of leather in terms of tensile strength, tear strength and grain bursting strength were comparable to that obtained by lime and sulphide based dehairing. The reduction in pollution load was also assessed by analyzing the waste streams of both control and experimental process. The results indicate that there was significant reduction in COD and sulfide. The BOD/COD ratio of the waste stream of experiment was found to be 0.70. This indicates that the degradability and treatability of the wastewater from experiment is improved.

**Example 57**

This example illustrates the process for unhairing of buffalo hides using crude protease from *Conidiobolus brefeldianus* MTCC 5185. The protease produced as described in example 6B was subjected to ammonium sulphate precipitation (90% saturation) and used for dehairing studies. Wet salted buffalo hides were taken, washed and soaked for 8 hours. After soaking, the weight of the hides was noted. The hides were treated in drums using 15% water and 3-4 % enzyme. A corresponding chemical based control process was also carried out using 150% water, 10% lime and 3% sodium sulfide. In the case experiment, the drum was run intermittently for 10 minutes for every hour. After six hours of running, the pelts were washed. The unhairing was 100%. In the case of control the drum was run for a day intermittently for 5 minutes for every hour. Next day the pelts were unhaired and the unhairing efficacy was 100%. The experimental pelts were clean and free from scud. The dehaired pelts were tanned and further processed into crust leathers and the quality of the crust leathers was also assessed. The quality of leather in terms of tensile strength, tear strength and grain bursting strength were comparable to that obtained by lime and sulphide method of dehairing. The reduction in pollution load was also assessed by analyzing the waste streams of both control and experimental process. The results indicate significant reduction in COD and sulfide with BOD/COD ratio of 0.74 in the case of experiment, which indicates that the degradability and treatability of the wastewater are enhanced.

**Example 58**

This example describes the application of protease in detergent formulation. A piece of white cloth was dipped in blood and air dried. The blood stained cloth was cut into four equal parts of 0.5g each and dipped in 2% formaldehyde for 2 min in 4 different petri plates and rinsed with water to remove excess formaldehyde. The pieces were dipped in 30ml of reaction mixture and incubated at 28-30°C for 30 min under different conditions: (a) control (only water) (b) Only detergent (c) Only crude protease (10 U) and (d) detergent + crude protease (10 U). The cloth pieces were rinsed 4 times with tap water. The washing performance was best where detergent and protease was used together. Blood clots were not removed with only detergent while protease alone was able to remove blood stain along with clots (Fig. 14).

**The main advantages of the present invention are the following**

1. The fungal strain is a new isolate.
2. The fungal strain described in the present invention is able to produce protease singly and in association with chitinase, lipase, chondrotinase, and keratinase.
3. These cocktail of enzymes have many industrial applications such as leather, textile, detergent and food industries.
4. The protease has already been evaluated for its applications in pre-tanning operations in leather industry.
5. The crude protease can dehair animal skins/hides without the requirement of any added chemicals like lime and sulphide.
6. The crude protease preparation has broad substrate specificity and can degrade variety of proteins such as albumin, hemoglobin, gelatin, keratins as required for applications in leather and detergent industries.
7. The crude protease can be used for degumming of silk.
8. Value added products like serecin peptides can be separated from waste liquor after enzymatic degumming with the protease.

**Claims**

**1.** A fungal strain *Conidiobolus brefeldianus* bearing accession number MTCC 5185.

2. A process for the preparation of one or more enzymes selected from the group consisting of protease, carbohydrase and lipase either singly or simultaneously from *Conidiobolus brefeldianus* bearing accession number MTCC 5185 depending on the carbon, nitrogen and inducer used, wherein said process comprises:

   a) growing the fungal strain *Conidiobolus brefeldianus* MTCC 5185 in a medium comprising a carbon, a nitrogen source and an inducer under aerobic conditions, pH ranging from 5.0 to 10, and temperatures ranging between 15° to 37°C, for periods ranging between 2 to 5 days;
   b) harvesting the medium and
   c) separating/extracting the enzyme in liquid phase by conventional methods.

3. The process according to claim 2, wherein said process comprises growing the *Conidiobolus* species in submerged conditions or
in solid state fermentation.

4. The process according to claim 2 wherein said proteases are selected from, but not limited to alkaline proteases, elastase, keratinase, and peptidase, alone or in combinations thereof.

5. The process according to claim 2 wherein said carbohydrases are selected from, but not limited to glycosidases, particularly glycanases, more particularly chitinase, laminarinase and chondroitinase alone or in combinations thereof.

6. The process according to claim 2 wherein said lipases comprise esterases.

7. The process according to claim 2, wherein the protease is active in the temperature range 30 to 60 degrees, stable at pH range of 3-12, preferably pH 7.

8. The process according to claim 2, wherein the protease is stable in presence of detergents, water miscible and water immiscible organic solvents.

9. The process according to claim 2, wherein the protease is stable up to 50°C.

10. The process according to claim 2, wherein the protease is active in the pH range of 6-11, and in presence of chelators, metal ions.

11. The process according to claim 2, wherein the crude and partially purified protease is active towards casein, albumin, haemoglobin, keratin, elastin-orcin, azocasein, azocoll, N-$\alpha$-benzoyl-DL-arginine-p-nitroanilide (BAPNA) and gelatin.

12. The process according to claim 2, wherein protease is inert to true collagen.

13. The process according to claim 2, wherein said lipase is active in the pH range 4 to 9 and in temperature range of 20 to 60°C.

14. The process according to claim 2, wherein said enzyme is in crude form, immobilized form, cell bound form, refined form, and purified form is subjected to processes selected from, but not limited to spray drying, freeze drying, filtration, concentration, refinement and purification.

15. The process according to claim 2, further comprising the use of at least one enzyme selected from protease, carbohydrase and lipase from *Conidiobolus* species, bearing accession number MTCC 5185, for dehairing operations in leather manufacture, recovery of silver from photographic film, degumming of silk, recovery of value added products from waste degumming liquor and detergent compositions.

## Patentansprüche

1. Pilzstamm *Conidiobolus brefeldianus* mit der Hinterlegungsnummer MTCC 5185.

2. Verfahren zur Herstellung von einem oder mehreren Enzymen, ausgewählt aus der Gruppe, bestehend aus Protease,

Carbohydrase und Lipase, entweder einzeln oder zusammen, von *Conidiobolus brefeldianus* mit der Hinterlegungsnummer MTCC 5185, abhängig von dem verwendeten Kohlenstoff, Stickstoff und Inducer, wobei das Verfahren umfasst:

a) Wachsenlassen des Pilzstammes *Conidiobolus brefeldianus* MTCC 5185 in einem Medium, umfassend eine Kohlenstoff-, eine Stickstoffquelle und einen Inducer, unter aeroben Bedingungen, einen pH im Bereich von 5,0 bis 10 und Temperaturen im Bereich zwischen 15 bis 37 °C, für Zeitperioden zwischen 2 bis 5 Tagen;
b) Ernten des Mediums, und
c) Trennen/Extrahieren des Enzyms in flüssiger Phase mit herkömmlichen Verfahren.

**3.** Verfahren nach Anspruch 2, wobei das Verfahren das Wachsenlassen der *Conidiobolus*-Spezies in untergetauchten Bedingungen oder in Festkörperfermentation umfasst.

**4.** Verfahren nach Anspruch 2, wobei die Proteasen ausgewählt sind aus alkalischen Proteasen, Elastase, Keratinase und Peptidase, alleine oder in Kombinationen davon, aber nicht hierauf beschränkt.

**5.** Verfahren nach Anspruch 2, wobei die Carbohydrasen ausgewählt sind aus Glycosidasen, insbesonderen Glykanasen, spezieller Chitinase, Laminarinase und Chondroitinase, alleine oder in Kombinationen davon, aber nicht hierauf beschränkt.

**6.** Verfahren nach Anspruch 2, wobei die Lipasen Esterasen umfassen.

**7.** Verfahren nach Anspruch 2, wobei die Protease im Temperaturbereich von 30 bis 60 °C aktiv und stabil in einem pH-Bereich von 3-12, bevorzugt pH 7, ist.

**8.** Verfahren nach Anspruch 2, wobei die Protease in der Anwesenheit von Detergentien, wassermischbaren und wasserunmischbaren organischen Lösungsmitteln stabil ist.

**9.** Verfahren nach Anspruch 2, wobei die Protease bis zu 50 °C stabil ist.

**10.** Verfahren nach Anspruch 2, wobei die Protease in dem pH-Bereich von 6-11 und in Anwesenheit von Chelatoren, Metallionen aktiv ist.

**11.** Verfahren nach Anspruch 2, wobei die krude und partiell aufgereinigte Protease gegenüber Casein, Albumin, Hämoglobin, Keratin, Elastin-Orcin, Azocasein, Azocoll, N-$\alpha$-Benzoyl-DL-Arginin-P-Nitroanilid (BAPNA) und Gelatine aktiv ist.

**12.** Verfahren nach Anspruch 2, wobei die Protease gegenüber echtem Collagen inert ist.

**13.** Verfahren nach Anspruch 2, wobei die Lipase im pH-Bereich von 4 bis 9 und im Temperaturbereich von 20 bis 60 °C aktiv ist.

**14.** Verfahren nach Anspruch 2, wobei das Enzym in kruder Form, immobilisierter Form, zellgebundener Form, raffinierter Form, und wobei die aufgereinigte Form Prozessen unterworfen wird, ausgewählt aus, aber nicht beschränkt auf Sprühtrocknung, Gefriertrocknung, Filtration, Konzentration, Raffination und Aufreinigung.

**15.** Verfahren nach Anspruch 2, weiterhin umfassend die Verwendung von wenigstens einem Enzym, ausgewählt aus Protease, Carbohydrase und Lipase, von *Conidiobolus* Spezies, mit der Hinterlegungsnummer MTCC 5185, für Enthaarungsschritte bei der Lederherstellung, der Wiedergewinnung von Silber von photographischem Film, Degummierung von Seide, Wiedergewinnung von Mehrwertprodukten aus Entgummierung aus Abwasserflüssigkeit vom Degummieren und Detergens-Zusammensetzungen.

## Revendications

**1.** Souche de champignon *Conidiobolus brefeldianus* ayant le numéro d'enregistrement MTCC 5185.

**2.** Procédé de préparation d'une ou de plusieurs enzymes choisies dans le groupe constitué par une protéase, une

carbohydrase et une lipase, une à la fois ou simultanément, à partir de *Conidiobolus brefeldianus* ayant le numéro d'enregistrement MTCC 5185 en fonction du carbone, de l'azote et de l'inducteur utilisés, dans lequel ledit procédé comprend :

a) la croissance de la souche de champignon *Conidiobolus brefeldianus* MTCC 5185 dans un milieu comprenant une source de carbone, une source d'azote et un inducteur dans des conditions aérobies, à un pH de 5,0 à 10 et à des températures allant de 15 °C à 37 °C pendant des périodes de 2 à 5 jours ;
b) la collecte du milieu et
c) la séparation/extraction de l'enzyme en phase liquide par des procédés classiques

**3.** Procédé selon la revendication 2, dans lequel ledit procédé comprend la croissance de l'espèce *Conidiobolus* dans des conditions submergées ou
par fermentation à l'état solide.

**4.** Procédé selon la revendication 2, dans lequel lesdites protéases sont choisies parmi, mais sans s'y limiter, les protéases alcalines, l'élastase, la kératinase et la peptidase, seules ou dans des combinaisons de celles-ci.

**5.** Procédé selon la revendication 2, dans lequel lesdites carbohydrases sont choisies parmi, mais sans s'y limiter, les glycosidases, en particulier les glycanases, plus particulièrement la chitinase, la laminarinase et la chondroïtinase, seules ou dans des combinaisons de celles-ci.

**6.** Procédé selon la revendication 2, dans lequel lesdites lipases comprennent les estérases.

**7.** Procédé selon la revendication 2, dans lequel la protéase est active dans la plage de températures allant de 30 à 60 degrés, stables dans une plage de pH allant de 3 à 12, de préférence à pH 7.

**8.** Procédé selon la revendication 2, dans lequel la protéase est stable en présence de détergents, des solvants organiques miscibles à l'eau et des solvants organiques non miscibles à l'eau

**9.** Procédé selon la revendication 2, dans lequel la protéase est stable jusqu'à 50 °C.

**10.** Procédé selon la revendication 2, dans lequel la protéase est active dans une plage de pH allant de 6 à 11, et en présence de chélateurs, d'ions métalliques.

**11.** Procédé selon la revendication 2, dans lequel la protéase brute et partiellement purifiée est active sur la caséine, l'albumine, l'hémoglobine, la kératine, l'élastine-orcine, l'azocaséine, l'azocoll, le N-$\alpha$-benzoyl-DL-arginine-p-nitroanilide (BAPNA) et la gélatine.

**12.** Procédé selon la revendication 2, dans lequel la protéase est inerte vis-à-vis du vrai collagène.

**13.** Procédé selon la revendication 2, dans lequel ladite lipase est active dans la plage de pH allant de 4 à 9 et dans une plage de température allant de 20 °C à 60 °C.

**14.** Procédé selon la revendication 2, dans lequel ladite enzyme est sous forme brute, sous forme immobilisée, sous une forme liée à une cellule, sous forme raffinée et sous forme purifiée, est soumise à des procédés choisis parmi, mais sans s'y limiter, un séchage par pulvérisation, une lyophilisation, une filtration, une concentration, un raffinage et une purification.

**15.** Procédé selon la revendication 2, comprenant en outre l'utilisation d'au moins une enzyme choisie parmi une protéase, une carbohydrase et une lipase issues de l'espèce *Conidiobolus,* ayant le numéro d'enregistrement MTCC 5185, pour des opérations d'épilage lors de la fabrication du cuir, la récupération d'argent à partir d'un film photographique, le dégommage de la soie, la récupération de produits à valeur ajoutée dans une liqueur résiduaire de dégommage et les compositions détergentes.

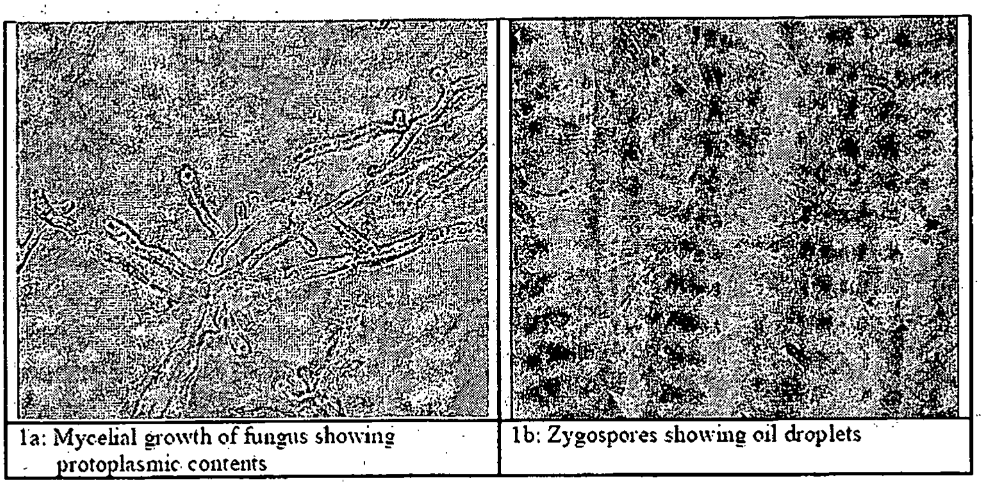

| 1a: Mycelial growth of fungus showing protoplasmic contents | 1b: Zygospores showing oil droplets |
|---|---|

Figure 1

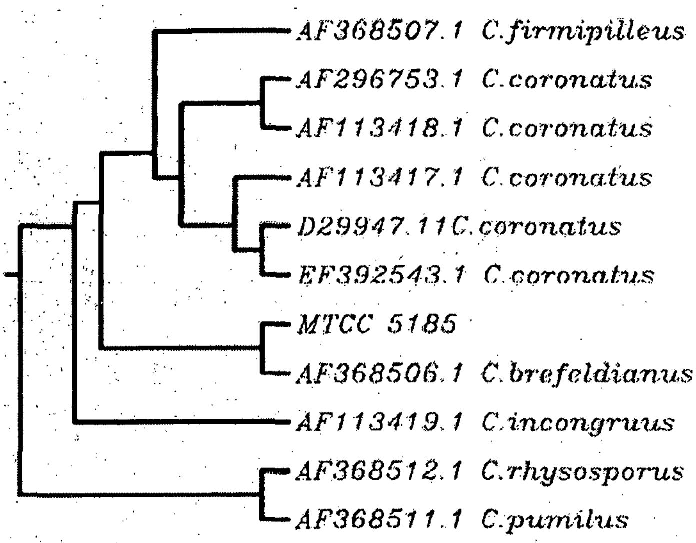
AF368507.1 C.firmipilleus
AF296753.1 C.coronatus
AF113418.1 C.coronatus
AF113417.1 C.coronatus
D29947.1 1C.coronatus
EF392543.1 C.coronatus
MTCC 5185
AF368506.1 C.brefeldianus
AF113419.1 C.incongruus
AF368512.1 C.rhysosporus
AF368511.1 C.pumilus

Figure 2

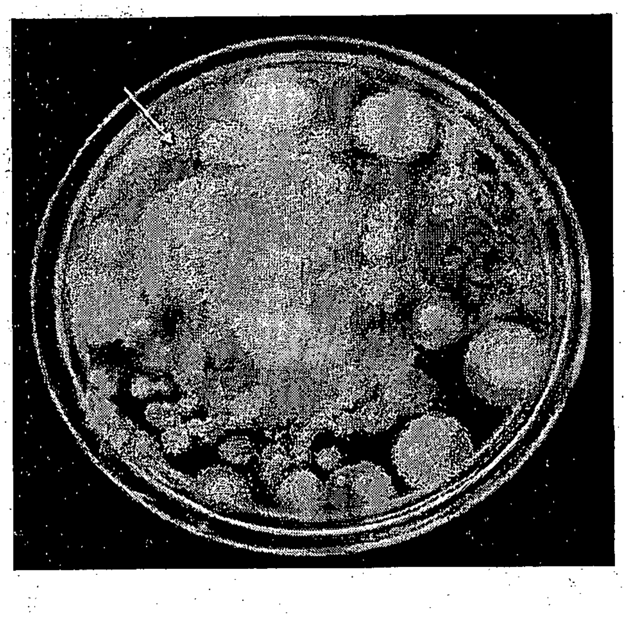

Figure 3

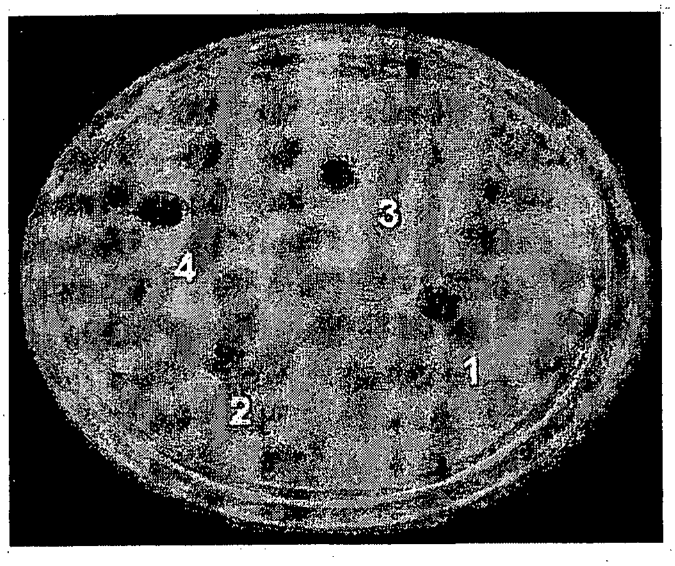


Figure 4

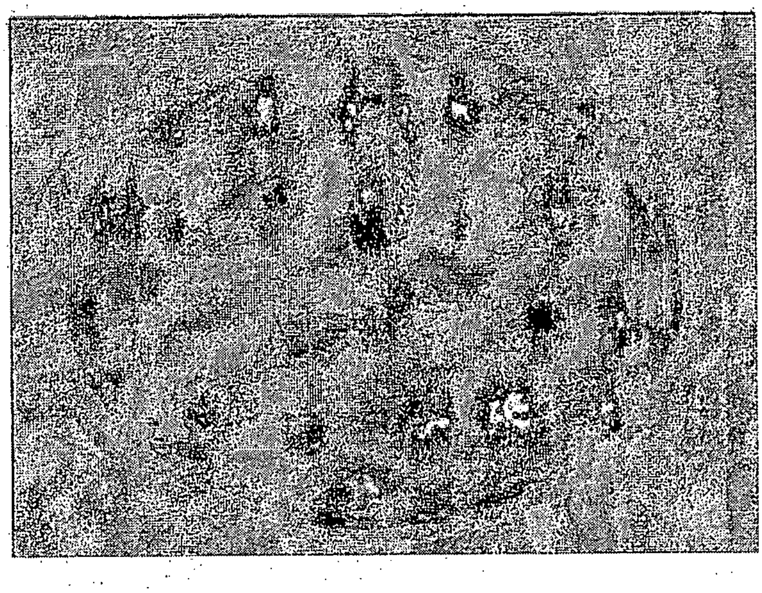

Figure 5

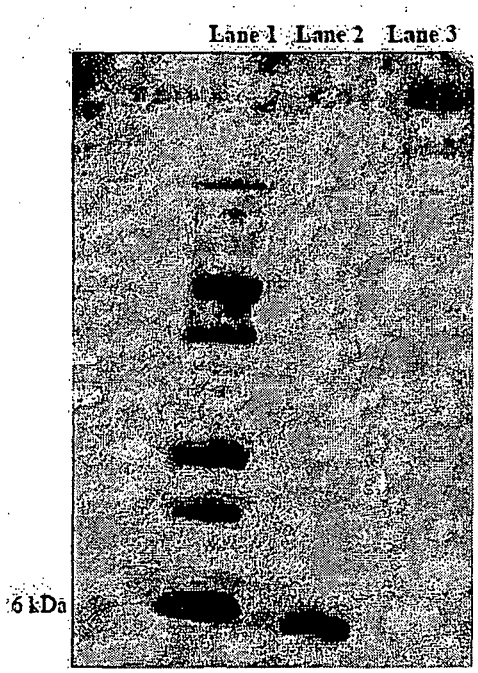


Lane-1: Markers; Lane 2: DGL (P); Lane 3: DGL (Control)

Figure 6

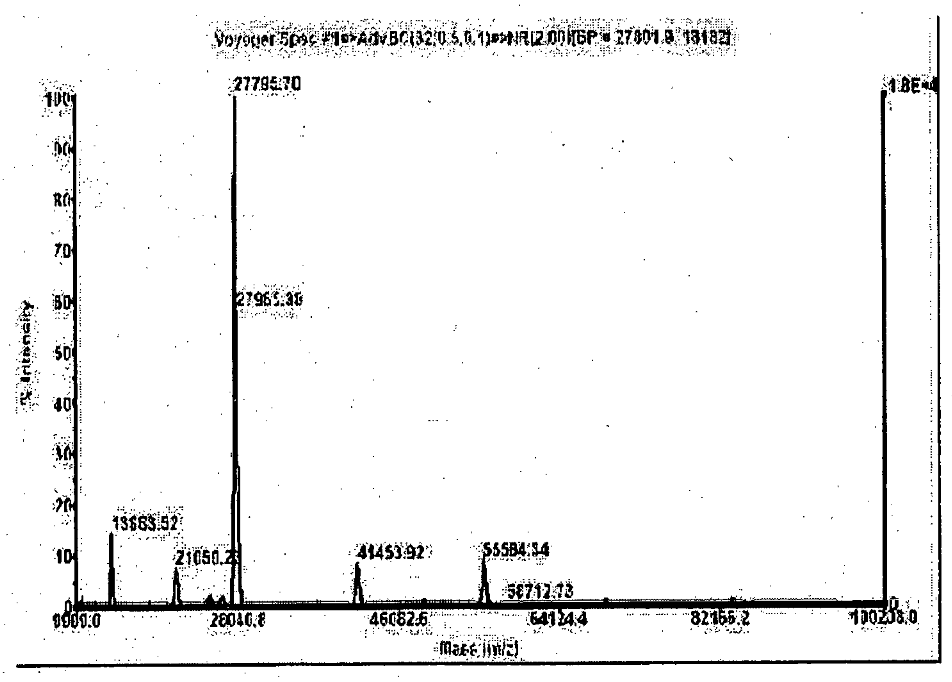
Voyager Spec #1=>AdvBC(32,0.5,0.1)=>NR(2.00)[BP = 27801.9, 18182]
27795.70
27965.30
13863.52
21050.23
41453.92
55584.34
56712.73
1.8E+4
% Intensity
Mass (m/z)
10000.0
28040.8
46082.6
64124.4
82166.2
100208.0

Figure 7

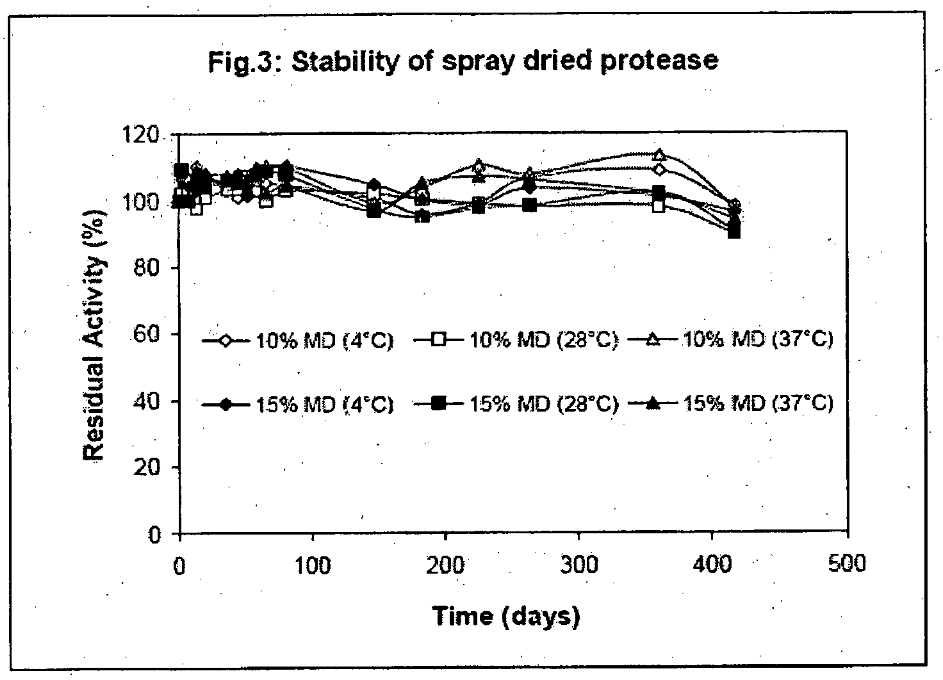
Fig.3: Stability of spray dried protease
Residual Activity (%)
120
100
80
60
40
20
0
0
100
200
300
400
500
Time (days)
10% MD (4°C)
10% MD (28°C)
10% MD (37°C)
15% MD (4°C)
15% MD (28°C)
15% MD (37°C)

Figure 8

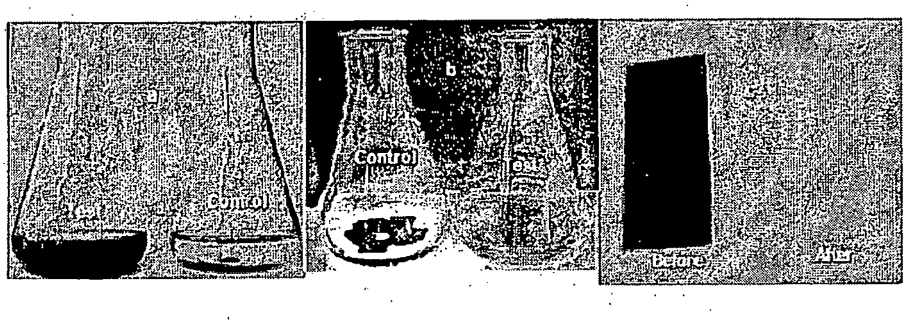


Figure 9

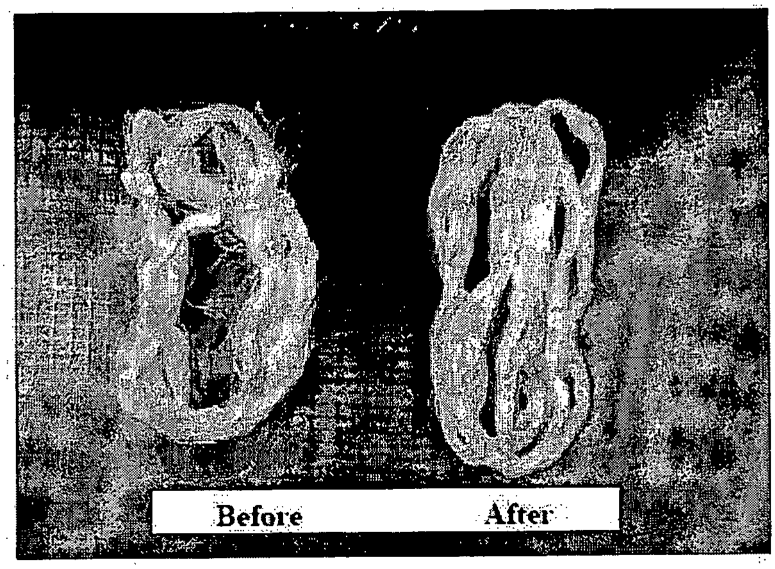


Figure 10

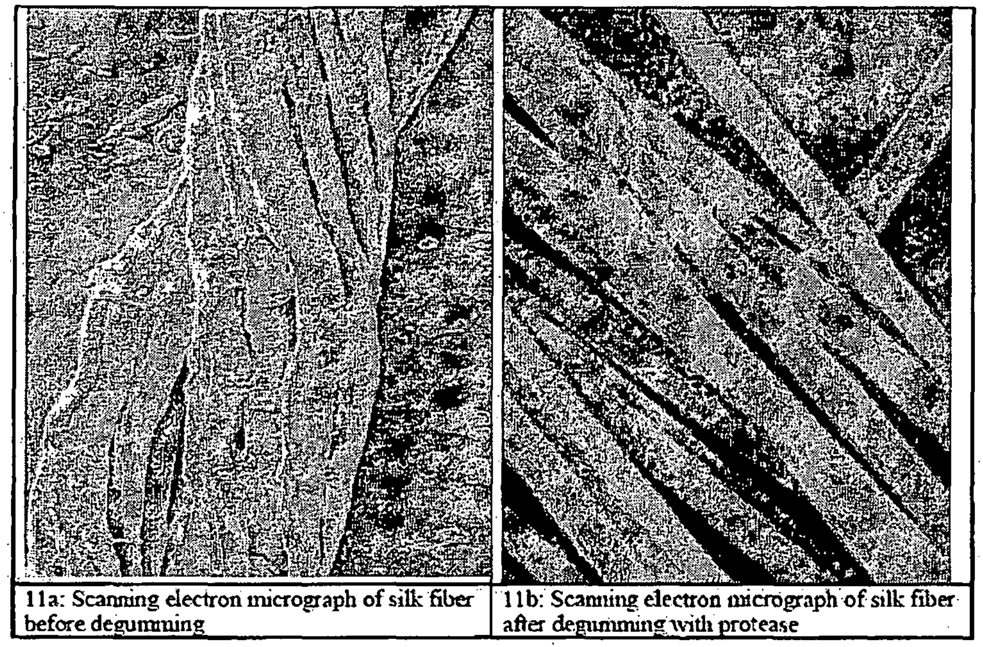

| | |
|---|---|
| 11a: Scanning electron micrograph of silk fiber before degumming | 11b: Scanning electron micrograph of silk fiber after degumming with protease |

Figure 11

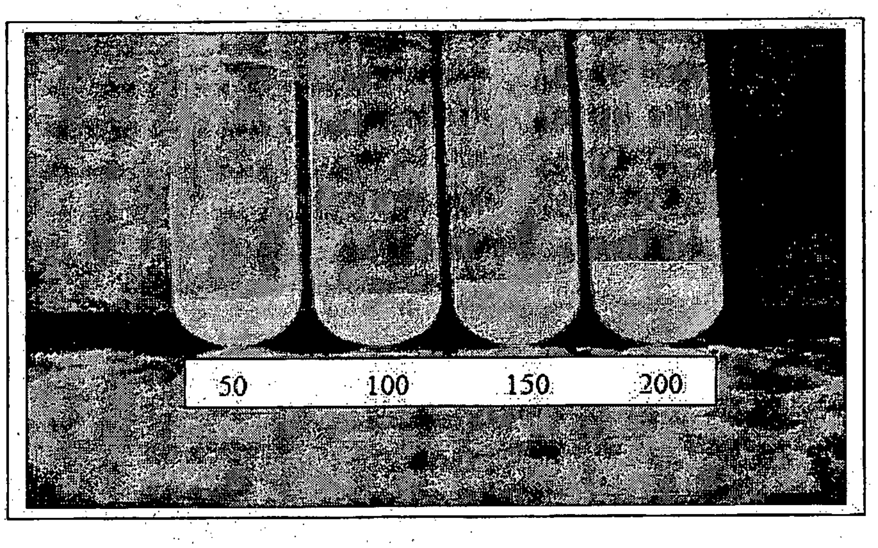


Figure 12

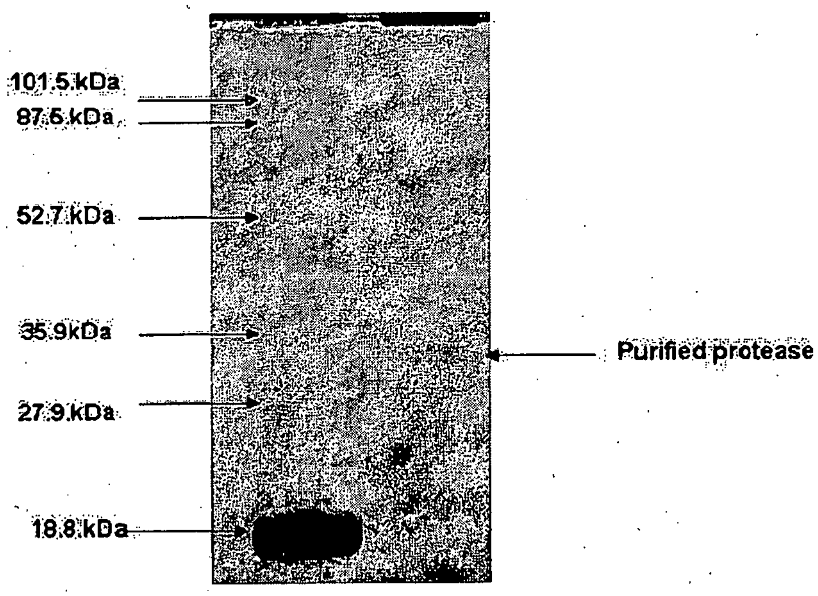
101.5 kDa
87.6 kDa
52.7 kDa
35.9kDa
27.9 kDa
18.8 kDa
Purified protease

Figure 13

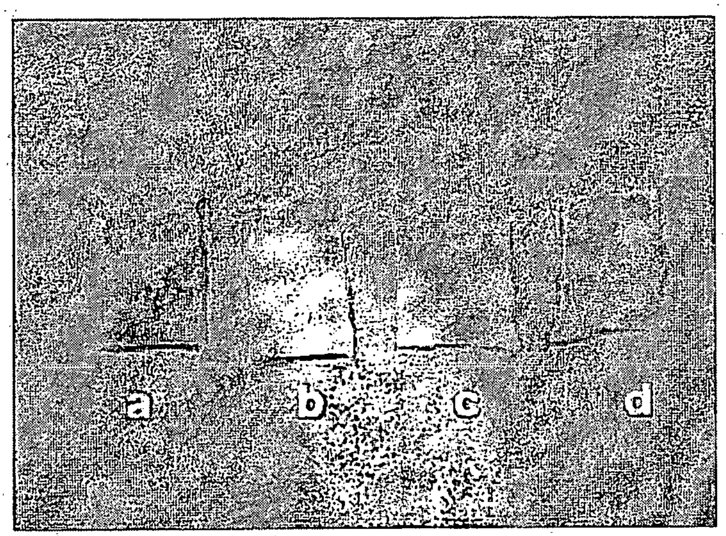


Figure 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6777219 B
- US 7186546 B


### Non-patent literature cited in the description


- **R. SEETA LAXMAN.** Optimization and scale up of production of alkaline protease from Conidiobolus coronatus. *Process Biochemistry,* September 2005, vol. 40 (9), 3152-3158
- **S. H. BHOSALE.** Thermostability of high-activity alkaline protease from Conidiobolus coronatus (NCL 86.8.20). *Enzyme and Microbial Technology,* 1995, vol. 17, 136-139
- **P. BERNFELD.** *Amylase: $\alpha$ & $\beta$, Methods in Enzymology,* 1955, vol. 1, 149
- **LAXMAN et al.** *Process Biochemistry,* 2005, vol. 40, 3152-3158
- **BRESSOLLIER et al.** *Applied Environmental Microbiology,* 1999, vol. 65 (6), 2570-2576